(19) Europäisches Patentamt
European Patent Office
Office européen des brevets

(11) EP 3 643 292 A1

(12) EUROPEAN PATENT APPLICATION

(43) Date of publication:
**29.04.2020 Bulletin 2020/18**

(21) Application number: **18202205.3**

(22) Date of filing: **24.10.2018**

(51) Int Cl.:
*A61K 8/34* (2006.01)
*A61K 8/41* (2006.01)
*A61Q 5/12* (2006.01)
*A61K 8/81* (2006.01)
*A61K 8/11* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **The Procter & Gamble Company**
**Cincinnati, OH 45202 (US)**

(72) Inventors:
- **OH, Hiroshi**
  **Cincinnati, Ohio 45202 (US)**
- **UEHARA (MATSUOKA), Nobuaki**
  **Singapore (SG)**
- **HALL, Dorothy**
  **Cincinnati, Ohio 45202 (US)**
- **NIJAKOWSKI, Timothy Roy**
  **Cincinnati, Ohio 45202 (US)**
- **TASSOS, Matthew Benjamin**
  **Cincinnati, Ohio 45202 (US)**
- **SMITH, Steven Daryl**
  **Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(54) **CONDITIONER COMPOSITIONS WITH INCREASED DEPOSITION OF POLYACRYLATE MICROCAPSULES**

(57) Described herein, a conditioner composition can help to increase the deposition and retention of benefit agent containing polyacrylate microcapsules onto hair. The conditioner composition includes a combination of polyacrylate microcapsules, wherein a block co-polymer is disposed on an outer surface of the polyacrylate microcapsules, deposition polymers, conditioner agents, and a carrier.

EP 3 643 292 A1

## Description

FIELD OF THE INVENTION

The present application generally relates to conditioner compositions containing polyacrylate microcapsules, wherein the polyacrylate microcapsules have increased deposition onto hair.

BACKGROUND OF THE INVENTION

Many of the conditioner products in the market today work to deliver benefits to hair by depositing benefit agents such as perfumes and conditioning agents onto the hair during conditioning. As a result, there is a desire to maximize the effectiveness of such benefit agents by increasing their delivery and retention onto hair.

In particular, benefit agents such as perfumes may delight the user by providing a freshness feeling and may serve as a signal to the user that the product may still be working or that the product is still present. Yet because of the volatility of many perfumes, a consumer may be unable to notice the perfume shortly after using the consumer product, potentially leading the user to believe the benefits are dissipating or have dissipated. Consequently, it may be also desirable to have technologies that improve the noticeability of perfumes in consumer products, especially after use of the consumer products.

Microcapsules have been used previously to encapsulate benefit agents such as perfumes in consumer products in order to provide longer lasting freshness benefits after use of the consumer product. Microcapsules typically contain the perfume until the capsule is fractured during use, thereby releasing the perfume to provide freshness benefits.

While these microcapsules are able to encapsulate a wide variety of benefit agents and deliver them to hair, it is still difficult to improve the retention and delivery efficiencies of such benefit agents. Such agents may be lost due to the agents' physical or chemical characteristics, may be washed off of the hair during conditioning, or may be incompatible with other compositional components already on the hair. Consumers today desire conditioning compositions that deposit and retain encapsulated benefit agents onto hair even after an extended period of time.

Accordingly, there is a need for a conditioner composition that provides an increased deposition of encapsulated benefit agents onto hair. In addition, there is a need for a polymer system that associates with microcapsule surfaces, and that when sheared, allows the encapsulated benefit agents being released. Furthermore, there is a need for a conditioner composition that provides an increased retention of encapsulated benefit agents onto the hair for an extended period of time.

SUMMARY OF THE INVENTION

A conditioner composition is provided and comprises:

(a) from 0.004% to 10% of polyacrylate microcapsules by weight of the conditioner composition, wherein the polyacrylate microcapsules comprise an outer surface, wherein the polyacrylate microcapsules comprise a shell material encapsulating a core material, said core material being disposed within said shell material, wherein said shell material comprises a polyacrylate polymer and said core material comprises a benefit agent; and wherein a block copolymer is disposed on the outer surface of the polyacrylate microcapsules, and wherein said block co-polymer has a formula:

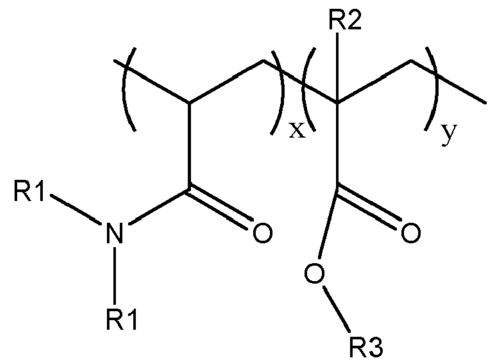

wherein

x and y are integers independently selected such that a molar ratio of monomer units represented by x to

monomer units represented by y is from 1.6:1 to 2.5:1;
each R1 is independently selected from the group consisting of H and $CH_3$;
each R2 is independently selected from the group consisting of H and $CH_3$; and
each R3 is independently a $C_1$-$C_{18}$ alkyl group;

wherein said block co-polymer has a number average molecular weight of at least 11 kDa according to the Molecular Weight Test Method as disclosed herein;
(b) from 0.05% to 8% of a deposition polymer by weight of the conditioner composition, wherein the deposition polymer is a copolymer comprising: a vinyl monomer (A) with a carboxyl group in the structure; and a vinyl monomer (B) expressed by the following formula (1):

$$CH_2=C(R^1)\text{-}CO\text{-}X\text{-}(Q\text{-}O)_r\text{-}R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q\text{-}O)_r\text{-}R^2$, the number of atoms bonded in a straight chain is 70 or less; and wherein the vinyl monomer (A) is contained at a level of from 10 mass% to 50 mass% based on the total mass of the copolymer, and the vinyl monomer (B) is contained at level of from 50 mass% to 90 mass% based on the total mass of the copolymer; and
(c) from 0.05% to 40 % of a conditioning agent, preferably from 0.5% to 30% of a conditioning agent, more preferably from 2% to 25% of a conditioning agent by weight of the conditioner composition, wherein the conditioning agent is selected from the group consisting of a cationic surfactant, a high melting point fatty compound, a silicone compound, and combinations thereof; and
(d) a carrier.

A method of making a conditioner composition is provided and comprises, preferably in that order, the steps of:

a) adding a deposition polymer to a conditioning agent to form a pre-conditioner composition, wherein the deposition polymer is a copolymer comprising: a vinyl monomer (A) with a carboxyl group in the structure; and a vinyl monomer (B) expressed by the following formula (1):

$$CH_2=C(R^1)\text{-}CO\text{-}X\text{-}(Q\text{-}O)_r\text{-}R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q\text{-}O)_r\text{-}R^2$, the number of atoms bonded in a straight chain is 70 or less; and wherein the vinyl monomer (A) is contained at a level of from 10 mass% to 50 mass% based on the total mass of the copolymer, and the vinyl monomer (B) is contained at level of from 50 mass% to 90 mass% based on the total mass of the copolymer,
wherein the conditioning agent is selected from the group consisting of a cationic surfactant, a high melting point fatty compound, a silicone compound, and combinations thereof; and a carrier;
b) adding polyacrylate microcapsules, wherein a block co-polymer is disposed on an outer surface of the polyacrylate microcapsules, and wherein said block co-polymer has a formula:

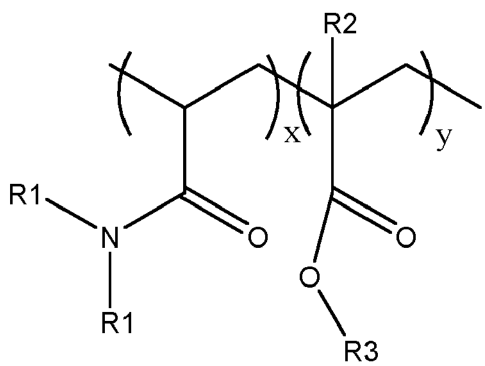

wherein

x and y are integers independently selected such that a molar ratio of monomer units represented by x to monomer units represented by y is from 1.6:1 to 2.5:1;
each R1 is independently selected from the group consisting of H and $CH_3$;
each R2 is independently selected from the group consisting of H and $CH_3$; and
each R3 is independently a $C_1$-$C_{18}$ alkyl group;

wherein said block co-polymer has a number average molecular weight of at least 11 kDa according to the Molecular Weight Test Method as disclosed herein; to the resulting pre-conditioner composition of step (a).

Alternatively, a method of making a conditioner composition is provided and comprises, preferably in that order, the steps of:

a) adding polyacrylate microcapsules, wherein a block co-polymer is disposed on an outer surface of the polyacrylate microcapsules, and wherein said block co-polymer has a formula

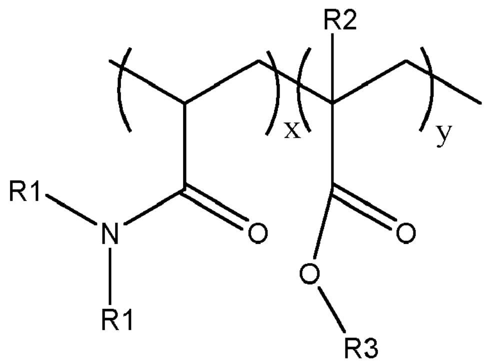

wherein

x and y are integers independently selected such that a molar ratio of monomer units represented by x to monomer units represented by y is from 1.6:1 to 2.5:1;
each R1 is independently selected from the group consisting of H and $CH_3$;
each R2 is independently selected from the group consisting of H and $CH_3$; and
each R3 is independently a $C_1$-$C_{18}$ alkyl group;

wherein said block co-polymer has a number average molecular weight of at least 11 kDa according to the Molecular Weight Test Method as disclosed herein; to a conditioning agent selected from the group consisting of a cationic surfactant, a high melting point fatty compound, a silicone compound, and combinations thereof; and a carrier, to form a pre-conditioner composition;
b) adding a deposition polymer to the resulting pre-conditioner composition of step (a), wherein the deposition polymer is a copolymer comprising: a vinyl monomer (A) with a carboxyl group in the structure; and a vinyl monomer (B) expressed by the following formula (1):

$$CH_2=C(R^1)\text{-}CO\text{-}X\text{-}(Q\text{-}O)_r\text{-}R^2 \quad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q\text{-}O)_r\text{-}R^2$, the number of atoms bonded in a straight chain is 70 or less; and wherein the vinyl monomer (A) is contained at a level of from 10 mass% to 50 mass% based on the total mass of the copolymer, and the vinyl monomer (B) is contained at level of from 50 mass% to 90 mass% based on the total mass of the copolymer.
Alternatively, a method of making a conditioner composition is provided and comprises, preferably in that order, the steps of:

a) combining polyacrylate microcapsules, wherein a block co-polymer is disposed on an outer surface of the polyacrylate microcapsules, and wherein said block co-polymer has a formula

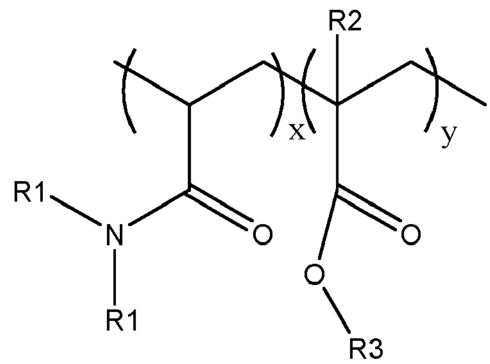

wherein

x and y are integers independently selected such that a molar ratio of monomer units represented by x to monomer units represented by y is from 1.6:1 to 2.5:1;
each R1 is independently selected from the group consisting of H and $CH_3$;
each R2 is independently selected from the group consisting of H and $CH_3$; and
each R3 is independently a $C_1$-$C_{18}$ alkyl group;

wherein said block co-polymer has a number average molecular weight of at least 11 kDa according to the Molecular Weight Test Method as disclosed herein; with a deposition polymer which is a copolymer comprising: a vinyl monomer (A) with a carboxyl group in the structure; and a vinyl monomer (B) expressed by the following formula (1):

$$CH_2=C(R^1)-CO-X-(Q-O)_r-R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4 which may also have a substitution group; r represents an integer of 2 to 15; and X represents an oxygen atom or an NH group; and, in the following structure $-(Q-O)_r-R^2$, the number of atoms bonded in a straight chain is 70 or less; and wherein the vinyl monomer (A) is contained at a level of from 10 mass% to 50 mass% based on the total mass of the copolymer, and the vinyl monomer (B) is contained at level of from 50 mass% to 90 mass% based on the total mass of the copolymer, to form a premix;
b) adding the premix of step (a) to a conditioning agent selected from the group consisting of a cationic surfactant, a high melting point fatty compound, a silicone compound, and combinations thereof; and a carrier.

Use of a deposition polymer in a conditioner composition comprising polyacrylate microcapsules, a conditioning agent and a carrier; for increasing the deposition of polyacrylate microcapsules onto hair for a period of at least 4 hours, preferably from 4 to 24 hours; or for providing a relatively long-lasting odor benefit for a period of at least 4 hours, preferably for a period of at least 24 hours, more preferably from 4 to 24 hours;
wherein the deposition polymer is the range from 0.05% to 8% by total weight of the conditioner composition, wherein the deposition polymer comprises a copolymer comprising: a vinyl monomer (A) with a carboxyl group in the structure; and a vinyl monomer (B) expressed by the following formula (1):

$$CH_2=C(R^1)-CO-X-(Q-O)_r-R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q-O)_r-R^2$, the number of atoms bonded in a straight chain is 70 or less; and wherein the vinyl monomer (A) is contained at a level of from 10 mass% to 50 mass% based on the total mass of the copolymer, and the vinyl monomer (B) is contained at level of from 50 mass% to 90 mass% based on the total mass of the copolymer; and
wherein a block co-polymer is disposed on an outer surface of the polyacrylate microcapsules, and wherein said block

co-polymer has a formula

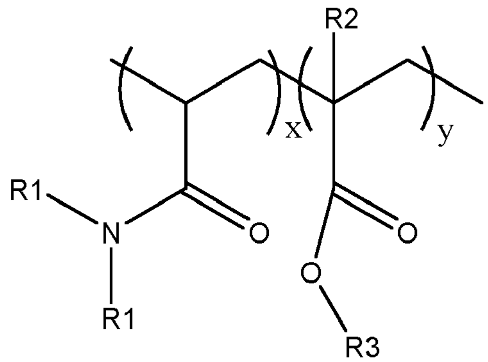

wherein

x and y are integers independently selected such that a molar ratio of monomer units represented by x to monomer units represented by y is from 1.6:1 to 2.5:1;
each R1 is independently selected from the group consisting of H and $CH_3$;
each R2 is independently selected from the group consisting of H and $CH_3$; and
each R3 is independently a $C_1$-$C_{18}$ alkyl group;

wherein said block co-polymer has a number average molecular weight of at least 11 kDa according to the Molecular Weight Test Method as disclosed herein.

DETAILED DESCRIPTION OF THE INVENTION

**Definitions of terms**

In this document, including in all embodiments of all aspects of the present invention, the following definitions apply unless specifically stated otherwise.

All percentages are by weight (w/w) of the respective composition, unless otherwise specified. All ratios or percentages are weight ratios or weight percentages unless specifically stated otherwise. "% wt." means percentage by weight. References to "parts" e.g. a mixture of 1 part X and 3 parts Y, is a ratio by weight. When more than one composition are used during a treatment, the total weight to be considered is the total weight of all the compositions applied on the hair simultaneously (i.e. the weight found "on head"), unless otherwise specified.

"QSP" or "q.s." means sufficient quantity for 100% or for 100g. "+/-" indicates the standard deviation. All ranges are inclusive and combinable. The number of significant digits conveys neither a limitation on the indicated amount nor on the accuracy of the measurement.

All measurements are understood to be made at 20°C and at ambient conditions, where "ambient conditions" means at 1 atmosphere (atm) of pressure and at 65% relative humidity, unless otherwise stated. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International.

Herein "min" means "minute" or "minutes". Herein "mol" means mole. Herein "g" following a number means "gram" or "grams". "Ex." means "example". All amounts as they pertain to listed ingredients are based on the active level ("solids") and do not include carriers or by-products that may be included in commercially available materials.

Herein, "comprising" means that other steps and other ingredients can be included in addition. "Comprising" encompasses the terms "consisting of' and "consisting essentially of'. The compositions, methods, and uses of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

The terms "include," "includes," and "including," as used herein are meant to be non-limiting.

Where amount ranges are given, these are to be understood as being the total amount of said ingredient in the composition, or where more than one species fall within the scope of the ingredient definition, the total amount of

all ingredients fitting that definition, in the composition.

For example, if the composition comprises from 1% to 5% fatty alcohol, then a composition comprising 2% stearyl alcohol and 1% cetyl alcohol and no other fatty alcohol, would fall within this scope.

The amount of each particular ingredient or mixtures thereof described hereinafter can account for up to 100% (or 100%) of the total amount of the ingredient(s) in the composition.

The term "substantially free of' as used herein means less than 1%, less than 0.8%, less than 0.5%, less than 0.3%, or less than an immaterial amount of by total weight of the composition.

The term "hair" as used herein means mammalian hair including scalp hair, facial hair and body hair, more preferably hair on the human head and scalp. Hair comprises hair fibers. "Hair shaft" means an individual hair strand and may be used interchangeably with the term "hair." As used herein the term "hair" to be treated may be "living" i.e. on a living body or may be "non-living" i.e. in a wig, hairpiece or other aggregation of non-living keratinous fibers. Mammalian, preferably human hair is preferred.

The term "consumer product" as used herein means conditioner products intended to be used or consumed in the form in which it is sold. Such products include but are not limited to products for and/or methods relating to treating hair including conditioning.

The term "conditioning agent" as used herein includes cationic surfactant, high melting point fatty compound, a silicone compound, and mixtures thereof.

The term "fluid" as used herein includes liquids and gels.

The terms "microcapsule" or "encapsulated benefit agents" as used herein refers to polyacrylate microcapsules.

The term "premix" as used herein refers to a mixture of polyacrylate microcapsules with deposition polymers before to be added to a conditioning agent.

The term "copolymer" as used herein for the deposition polymer refers to a polymer derived from two or more polymerizable monomers. When used in generic terms, the term "copolymer" is also inclusive of more than two distinct monomers, for example, terpolymers. The term "copolymer" as used herein for the block co-polymer refers to a polymer derived from two or more polymerizable monomers, preferably two polymerizable monomers.

The term "cosmetically acceptable" as used herein means that the compositions, or components described are suitable for use in contact with human keratinous tissue, especially hair without undue toxicity, incompatibility, instability, allergic response, and the like. All compositions described herein which have the purpose of being directly applied to keratinous tissue are limited to those being cosmetically acceptable.

The term "mixtures" as used herein is meant to include a simple combination of materials and any compounds that may result from their combination.

The term "molecular weight" or "M.Wt." as used herein refers to the weight average molecular weight unless otherwise stated. The weight average molecular weight of the deposition polymer can be measured by gel permeation chromatography. However, the number average molecular weight of the block co-polymer is measured according to the MOLECULAR WEIGHT TEST METHOD as disclosed herein.

**Benefits of polyacrylate microcapsules and deposition polymers**

Consumers desire conditioner compositions that deposit and retain encapsulated benefit agents onto their hair during the conditioning process. Traditionally, a variety of approaches have been employed to improve deposition of microcapsules, including (1) using specific block copolymers to covalently bind to the microcapsules, and (2) using cationic water-soluble polymers to coat the microcapsules in order to increase the affinity of the microcapsules to the substrate of interest. However, it is desired to have improved deposition over the traditional approaches.

It has been surprisingly found that when polyacrylate microcapsules are modified with a block co-polymer having a formula:

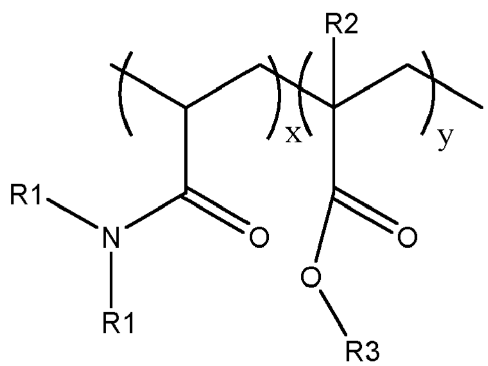

wherein

x and y are integers independently selected such that a molar ratio of monomer units represented by x to monomer units represented by y is from 1.6:1 to 2.5:1;
each R1 is independently selected from the group consisting of H and $CH_3$;
each R2 is independently selected from the group consisting of H and $CH_3$; and
each R3 is independently a $C_1$-$C_{18}$ alkyl group;

wherein said block co-polymer has a number average molecular weight of at least 11 kDa according to the Molecular Weight Test Method as disclosed herein, it results in an even more improved delivery efficiency of the encapsulated benefit agents to hair such as an improved deposition of the polyacrylate microcapsules onto keratinaceous hair surfaces, and thus an increased retention of onto hair. Also, these properties result in improved olfactive performance as illustrated by the experimental part hereinbelow.

It is believed that polyacrylate microcapsules modified by the block co-polymer as defined above and disposed on an outer surface of the polyacrylate microcapsules, and the addition of a deposition polymer to the conditioner composition can provide a synergistic effect on improving the deposition of polyacrylate microcapsules onto hair. The deposition polymer as described hereinafter can render the conditioner composition, preferably the gel matrix of the conditioner composition relatively more hydrophobic and bigger in terms of size. Then, the polyacrylate microcapsules modified by the block co-polymer are relatively more viscoelastic. Consequently, the polyacrylate microcapsules modified by the block co-polymer can strongly adhere to the hydrophobic conditioner composition, preferably the gel matrix to be retained onto the hair surface after treating the hair with the conditioner composition.

It is believed that the conditioner composition comprising polyacrylate microcapsules modified with the block co-polymer, along with a deposition polymer as defined more in details hereinbelow can help to deliver a higher deposition rate of polyacrylate microcapsules than conditioner compositions containing only polyacrylate microcapsules modified with the block co-polymer; or only unmodified polyacrylate microcapsules along with a deposition polymer even over an extended period of time.

## MICROCAPSULES

A conditioner composition comprises a polyacrylate microcapsule, preferably a plurality of polyacrylate microcapsules. The conditioner composition comprises from 0.004% to 10%, preferably from 0.01% to 8%, more preferably from 0.1% to 5%, or even more preferably from 0.25% to 3% of polyacrylate microcapsules by weight of the conditioner composition. The polyacrylate microcapsules comprise an outer surface. The polyacrylate microcapsules comprise a core material and a shell material encapsulating the core material which is disposed within the shell material. The shell material comprises a polyacrylate polymer and the core material comprises a benefit agent. A block co-polymer is disposed on the outer surface of the polyacrylate microcapsules. The block co-polymer has the formula and is defined as set out hereinbefore. The polyacrylate microcapsules may comprise an anionic emulsifier.

Preferred microcapsules comprising a shell material comprising polyacrylate material are described in detail in U.S. Application Serial No. 62/206,971 (Case 13998P).

The polyacrylate microcapsules will typically have a volume weighted median particle size from 2 microns to 80 microns, preferably from 3 microns to 60 microns. The volume weighted median particle size of the microcapsules may be more preferably from 5 microns to 45 microns, even more preferably from 8 microns to 30 microns. The volume weighted median particle size of the microcapsules is determined according to the Volume Weighted Median Particle Size Test Method hereinbelow.

### Shell material

The shell material comprises a polyacrylate polymer. The shell material may comprise from 50% to 100%, preferably from 70% to 100%, more preferably from 80% to 100%, by weight of the shell material, of a polyacrylate polymer.

The shell material may optionally further comprise polyvinyl alcohol. The shell material may comprise from 0.5% to 40%, preferably from 0.5% to 20%, more preferably from 0.5% to 10%, even more preferably from 0.8% to 5%, by weight of the shell material, of polyvinyl alcohol.

The polyacrylate polymer of the shell material can be derived from a material that comprises one or more multifunctional acrylate moieties. Preferably the multifunctional acrylate moiety may be selected from group consisting of tri-functional acrylate, tetra-functional acrylate, penta-functional acrylate, hexa-functional acrylate, hepta-functional acrylate, and mixtures thereof.

The polyacrylate polymer may optionally comprise a moiety selected from the group consisting of an amine acrylate moiety, a methacrylate moiety, a carboxylic acid acrylate moiety, a carboxylic acid methacrylate moiety, and

combinations thereof.

The polyacrylate polymer may be derived from a material that comprises one or more multifunctional acrylate and/or a material that comprises one or more methacrylate moieties, wherein the ratio of material that comprises one or more multifunctional acrylate moieties to material that comprises one or more methacrylate moieties is from 999:1 to 6:4, preferably from 99:1 to 8:1, more preferably from 99:1 to 8.5:1.

The polyacrylate polymer of the shell material may preferably comprise a cross-linked polyacrylate polymer.

The polyvinyl alcohol of the shell material, when present, may preferably have one or more of the following properties:

a hydrolysis degree from 55% to 99%, preferably from 75% to 95%, more preferably from 85% to 90%, even more preferably from 87% to 89%;

a viscosity of from 40 mPa.s to 80 mPa.s (40 cps to 80 cps), preferably from 45 mPa.s to 72 mPa.s (45 cps to 72 cps), more preferably from 45 mPa.s to 60 mPa.s (45 cps to 60 cps), even more preferably from 45 mPa.s to 55 mPa.s (45 cps to 55 cps) in 4% water solution at 20°C;

a degree of polymerization of from 1500 to 2500, preferably from 1600 to 2200, more preferably from 1600 to 1900, even more preferably from 1600 to 1800;

a weight average molecular weight of from 130 000 to 204 000, preferably from 146 000 to 186 000, more preferably from 146 000 to 160 000, even more preferably from 146 000 to 155 000; and/or

a number average molecular weight of from 65 000 to 110 000, preferably from 70 000 to 101 000, more preferably from 70 000 to 90 000, even more preferably from 70 000 to 80 000.

The test methods for determining the above properties of polyvinyl alcohol are described in detail in U.S. Application Serial No. 62/206,971 (Case 13998P), and are set out in the Test Methods section hereinbelow.

Core material

The core material disposed within the shell material of the polyacrylate microcapsule comprises a benefit agent. The core material can optionally further comprise a partitioning modifier.

The core material may comprise from 6% to 99.9% of a benefit agent by total weight of the core, preferably from 10% to 90% of a benefit agent by total weight of the core, more preferably from 35% to 85% of a benefit agent by total weight of the core, even more preferably from 60% to 75% of a benefit agent by total weight of the core.

Benefit agents

Benefit agents useful as core material of the polyacrylate microcapsules are generally liquid in form at 25°C. The benefit agent may preferably be a hydrophobic benefit agent such as perfume. Such hydrophobic benefit agents may be typically oils.

Suitable benefit agents can include perfumes, brighteners, insect repellants, silicones, waxes, flavors, vitamins, fabric softening agents, skin care agents, enzymes, perfume delivery system; conditioning agents, moisturizers, anti-bacterial agents, anti-microbial agents, thickeners, sensates, attractants, dyes, pigments, bleaches and mixtures thereof.

The benefit agent may preferably comprise perfumes, brighteners, enzymes, perfume delivery system; conditioning agents, moisturizers, anti-microbial agents, thickeners, sensates, attractants, dyes, pigments, bleaches and mixtures thereof.

The benefit agent may more preferably comprise perfume. The one or more perfumes may be selected from any perfume or perfume chemical suitable for topical application to the skin and/or hair and suitable for use in personal care compositions, preferably in conditioner compositions.

The perfume of the core material of the polyacrylate microcapsules may comprise fragrance components having a boiling point of greater than 250°C at 1 bar pressure in a weight proportion of less than 65%, preferably from 35% to 65% by weight of the total weight of fragrance components.

Alternatively, the perfume of the core material of the polyacrylate microcapsules may comprise fragrance components having a boiling point of greater than 250°C at 1 bar pressure in a weight proportion of greater than 65%, preferably from 65% to 85%, more preferably from 70% to 90% by weight of the total weight of fragrance components.

The perfume may be selected from high impact accord fragrance components having a ClogP of greater than 2 and odor detection thresholds of less than or equal to 50 parts per billion (ppb).

The polyacrylate microcapsules useful herein are those releasing the benefit agents for a period of time after initial application. Potential trigger mechanisms for release of the encapsulated benefit agents may include, but are not limited to, mechanical forces, dehydration, light, pH, temperature, hydrolysis, or even changes in ionic strength.

The conditioner composition may also comprise a non-encapsulated perfume. The non-encapsulated perfume

may comprise fragrance components having a boiling point of greater than 250°C at 1 bar pressure in a weight proportion of greater than 65%, preferably from 65% to 85%, more preferably from 70% to 90% by weight of the total weight of fragrance components. Alternatively, the non-encapsulated perfume may comprise fragrance components having a boiling point of less than 250°C at 1 bar pressure in a weight proportion of less than 65%, preferably from 35% to 65% by weight of the total weight of fragrance components.

The non-encapsulated perfume may be used to provide a first boost of fragrance or to provide another sustaining perfume experience over an extended period of time for the consumer.

The perfume of the core material of the polyacrylate microcapsules may preferably comprise fragrance components having a boiling point of greater than 250°C at 1 bar pressure in a weight proportion of less than 65%, preferably from 35% to 65% by weight of the total weight of fragrance components. In addition, the conditioner composition may preferably comprise a non-encapsulated perfume which comprises fragrance components having a boiling point of greater than 250°C at 1 bar pressure in a weight proportion of greater than 65%, preferably from 65% to 85%, more preferably from 70% to 90% by weight of the total weight of fragrance components.

In that case, by selecting a non-encapsulated perfume that is rich in fragrance components having a boiling point of above 250°C, the release of fragrance still is a burst, but a burst over an extended period of time compared with a perfume that is rich in fragrance components having a boiling point of at or below 250°C. In addition, by selecting a blend of fragrance components that is lean in components having a boiling point of above 250°C for encapsulation, it is possible for the perfume that is released from the polyacrylate microcapsules to be perceived more quickly than if the encapsulated blend were rich in components having a boiling point of above 250°C.

Partitioning modifier

When the core material of the polyacrylate microcapsule is an oil, such as perfume oil, the properties inherent to the oil may play a role in determining how much, how quickly, and how permeable the resultant shell material of the polyacrylate microcapsule will be when established at the oil/water interface. For example, when the oil of the core material includes highly polar materials, such materials may reduce the diffusion of the monomers and polymers to the oil/water interface, potentially resulting in a relatively thin and highly permeable polymeric shell material, which can lead to an inferior microcapsule. Incorporating a partitioning modifier to adjust the polarity of the core may alter the partitioning coefficient of the polar materials, allowing for the establishment of a thicker, more stable shell material of the microcapsule.

Suitable non-limiting examples of partitioning modifiers are described in detail in US Application Publication No. 2011/0268802. Preferred partitioning modifiers as part of the core material of the present polyacrylate microcapsules may be selected from the group consisting of vegetable oil, modified vegetable oil, isopropyl myristate, propan-2-yl tetradecanoate, and mixtures thereof. Suitable vegetable oils may be selected from the group consisting of castor oil, soybean oil, and mixtures thereof. Suitable modified vegetable oils may be selected from the group consisting of esterified vegetable oil, brominated vegetable oil, and mixtures thereof. Preferred partitioning modifiers may be selected from the group consisting of isopropyl myristate, propan-2-yl tetradecanoate, and mixtures thereof.

**Anionic Emulsifier**

The polyacrylate microcapsules may comprise an anionic emulsifier.

The addition of an anionic emulsifier forms a microstructure with a specified deposition polymer at the external surface of the polyacrylate microcapsules, i.e., the anionic emulsifier is at least a part of the external surface of the microcapsules, or is physically or chemically bound to the external surface of the microcapsules. Such physical bindings include, for example, hydrogen bonding, ionic interactions, hydrophobic interactions, and electron transfer interactions. Such chemical bindings include, for example, covalent bindings such as covalent grafting and crosslinking.

The anionic emulsifier may be present at a level by weight of from 0.1% to 40%, preferably from 0.5% to 10%, more preferably from 0.5% to 5%, by weight of the polyacrylate microcapsule.

The anionic emulsifier and the polyacrylate microcapsule may be mixed such that the weight ratio of the anionic emulsifier to the polyacrylate microcapsule is from 1.0 : 40 to 0.5 : 5, preferably from 1.0 : 30 to 1.0 : 15.

A variety of anionic emulsifiers can be used in the conditioner compositions as described below. The anionic emulsifiers may include, by way of illustrating and not limitation, water-soluble salts of alkyl sulfates, alkyl ether sulfates, alkyl isethionates, alkyl carboxylates, alkyl sulfosuccinates, alkyl succinamates, alkyl sulfate salts such as sodium dodecyl sulfate, alkyl sarcosinates, alkyl derivatives of protein hydrolyzates, acyl aspartates, alkyl or alkyl ether or alkylaryl ether phosphate esters, sodium dodecyl sulphate, phospholipids or lecithin, or soaps, sodium, potassium or ammonium stearate, oleate or palmitate, alkylarylsulfonic acid salts such as sodium dodecylbenzenesulfonate, sodium dialkylsulfosuccinates, dioctyl sulfosuccinate, sodium dilaurylsulfosuccinate, poly(styrene sulfonate) sodium salt, isobutylene-maleic anhydride copolymer, gum arabic, sodium alginate, carboxymethylcellulose, cellulose sulfate and pectin, poly(styrene sulfonate), isobutylene-maleic anhydride copolymer, gum arabic, carrageenan, sodium alginate, pectic acid, tragacanth

gum, almond gum and agar; semi-synthetic polymers such as carboxymethylcellulose, sulfated cellulose, sulfated methylcellulose, carboxymethyl starch, phosphated starch, lignin sulfonic acid; and synthetic polymers such as maleic anhydride copolymers (including hydrolyzates thereof), polyacrylic acid, polymethacrylic acid, acrylic acid butyl acrylate copolymer or crotonic acid homopolymers and copolymers, vinylbenzenesulfonic acid or 2-acrylamido-2-methylpropanesulfonic acid homopolymers and copolymers, and partial amide or partial ester of such polymers and copolymers, carboxymodified polyvinyl alcohol, sulfonic acid-modified polyvinyl alcohol and phosphoric acid-modified polyvinyl alcohol, phosphated or sulfated tristyrylphenol ethoxylates.

**Process of making polyacrylate microcapsules**

Suitable processes for making microcapsules comprising a shell material comprising polyacrylate polymer are described in detail in US9186642, US2011/0269657A1, US9221028, US2011/0268778A1, and US9162085.

The block co-polymer may be added to the polyacrylate microcapsules by mixing the block co-polymer with the microcapsules using a conventional mixing device, such as a spatula, in a conventional mixing container, such as a glass jar. After initial mixing, the mixture may be further mixed for several hours in a conventional shaker device at room temperature. On a commercial scale, the block co-polymer can be added to the polyacrylate microcapsules via conventional, commercial-scale mixing equipment.

The resulting block co-polymer-coated microcapsules can be combined with consumer product adjunct ingredients when the polyacrylate microcapsules are in one or more forms, including slurry form, neat particle form, and spray dried particle form. The polyacrylate microcapsules may be combined with the consumer product adjunct ingredients by methods that include mixing and/or spraying.

**BLOCK CO-POLYMER**

The block co-polymer of the conditioner composition has a formula:

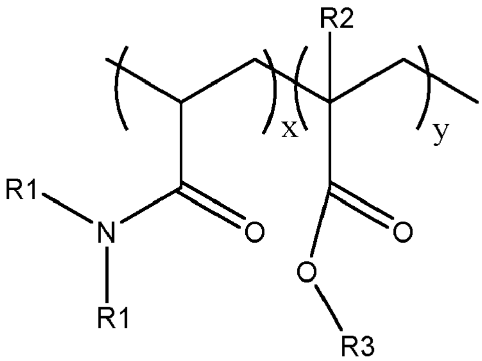

wherein

x and y are integers independently selected such that a molar ratio of monomer units represented by x to monomer units represented by y is from 1.6:1 to 2.5:1;
each R1 is independently selected from the group consisting of H and $CH_3$, preferably $CH_3$;
each R2 is independently selected from the group consisting of H and $CH_3$, preferably H; and
each R3 is independently a $C_1$-$C_{18}$ alkyl group, preferably a $C_1$-$C_8$ alkyl group, preferably a $C_1$-$C_4$ group, or preferably a $C_3$ alkyl group.

The block co-polymer of the conditioner composition may be a block co-polymer comprising monomers selected from the group consisting of acrylamide ("AAM"), dimethyl acrylamide ("DMAA"), n-alkylacrylate wherein n-alkyl is a $C_1$-$C_{18}$ alkyl group, preferably a $C_1$-$C_8$ alkyl group, preferably a $C_1$-$C_4$ group, or preferably a $C_3$ alkyl group ("AA"), and combinations thereof.

As further options, it is believed the effectiveness of the block co-polymer as a coating in improving the deposition of the polyacrylate microcapsules onto the hair fibers being treated with the conditioner composition comprising the deposition polymer may be affected by the number average molecular weight of the block co-polymer (as measured according to the MOLECULAR WEIGHT TEST METHOD herein), and the molar ratio of the monomer units represented by x to the monomer units represented by y.

If the number average molecular weight and/or molar ratio is too high, the block co-polymers may tend to be

insufficiently soluble in water. If the number average molecular weight and/or molar ratio are too low, the block co-polymer may tend to insufficiently form a viscoelastic gel on the surface of the coated polyacrylate microcapsules. The effectiveness of the block co-polymer as a coating can also be affected by the Water Uptake Value of the block co-polymer (as measured by the WATER UPTAKE VALUE TEST METHOD herein), which relates to the gelling capacity of the block co-polymer.

The number average molecular weight of the block co-polymer can be determined according to the MOLECULAR WEIGHT TEST METHOD hereinbelow. The block co-polymer of the conditioner composition may have a number average molecular weight of at least 11 kDa (kilodaltons), preferably from 11 to t 45 kDa, more preferably from 15 to 43 kDa, or even more preferably from 20 to 42 kDa.

Surface charge of the block co-polymer may be typically nonionic. The block co-polymer may be generally coated on the outer surface of the polyacrylate microcapsules due to a favored adhesion energy between two surfaces. The block co-polymer may tend to adhere to the outer surface of the polyacrylate microcapsules to form a deformable viscous gel layer.

The block co-polymers may be hydrophobic gels. These hydrophobic gels may tend to more effectively deposit and adhere to the treated hair fibers, thereby increasing the deposition of the block co-polymer-coated microcapsules versus microcapsules that are not coated with block co-polymer.

The block co-polymer may be combined with the polyacrylate microcapsules, thereby becoming disposed on the outer surface of the polyacrylate microcapsules, before the polyacrylate microcapsules are combined with the conditioner composition adjunct ingredients to form the conditioner composition.

The block co-polymer may be preferably incorporated in the conditioner composition in an amount of from 0.01% to 8%, preferably from 0.05% to 5%, more preferably from 0.1% to 3%, even more preferably 0.5% to 1.5%, by weight of the solid polyacrylate microcapsules. The total weight of the solid polyacrylate microcapsules means herein the total weight of the polyacrylate microcapsules as dried and not the total weight of the slurry of the polyacrylate microcaspules.

The block co-polymer of the conditioner composition may preferably have a Water Uptake Value, as measured by the WATER UPTAKE VALUE TEST METHOD herein, of at least 2 g/g, preferably from 3 to 50 g/g, more preferably from 3 to 25 g/g, even more preferably from 3 to 20 g/g, or most preferably from 3 to 15 g/g.

A preferred block co-polymer may have the formula above wherein x and y are integers selected such that the molar ratio of the monomer units represented by x to the monomer units represented by y is 1.65, R1 is $CH_3$, R2 is H, and R3 is a $C_3$ alkyl group. Such a preferred block co-polymer may have a number average molecular weight of 42 kDa, as measured by the MOLECULAR WEIGHT TEST METHOD herein, and a Water Uptake Value of 8, as measured by the WATER UPTAKE VALUE TEST METHOD herein. Such a preferred block co-polymer may be poly(N,N-dimethylacrylamide)-poly(n-butylacrylate).

The block co-polymer of the conditioner composition may be made according to the following general procedure. The desired n-alkylacrylate monomer may be added to a reaction vessel with a solvent (e.g. chlorobenzene), polymerization reagent (e.g. 2-(dodecylthiocarbonothioylthio)-2-methylpropionic acid), and initiator (e.g. 2,2'-azobis(2-methylpropionitrile)). The reaction vessel may be sparged with an inert gas (e.g. nitrogen) to remove oxygen from the system and maintain an inert gas atmosphere in the reaction vessel. The contents of the reaction vessel may be heated to an elevated temperature (e.g. 65°C). The contents of the reaction vessel may be maintained at elevated temperature for several hours (e.g. 24 hours). The resulting poly(n-alkylacrylate) solution is then precipitated in a solvent (e.g. cold hexane). The precipitate may be isolated and dried.

## DEPOSITION POLYMER

The conditioner composition comprises a deposition polymer. The deposition polymer is included at a level by weight of the conditioner composition of, from 0.05% to 8%, preferably from 0.1% to 5%, more preferably from 0.2% to 3.5%, by weight of the conditioner composition.

The deposition polymer useful herein is a copolymer comprising: a vinyl monomer (A) with a carboxyl group in the structure; and a vinyl monomer (B) expressed by the following formula (1):

$$CH_2=C(R^1)\text{-}CO\text{-}X\text{-}(Q\text{-}O)_r\text{-}R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q-O)_r-R^2$, the number of atoms bonded in a straight chain, i.e. the number of atoms bonded linearly to each other in the structure of $-(Q-O)_r-R^2$, is 70 or less.

In the formula (1), r may represent from 3 to 12. In the formula (1), X may represent an oxygen atom.

Vinyl Monomer (A)

The deposition polymer which is a copolymer contains a vinyl monomer (A) having a carboxyl group in the structure. The copolymer may contain one type of the vinyl monomer (A), or may contain two or more types of the vinyl monomer (A). The vinyl monomer (A) may be preferably anionic.

The vinyl monomer (A) is contained at a level of from 10 mass% based on the total mass of the copolymer, preferably from 15 mass%, more preferably 20 mass % or higher, and even more preferably 25 mass% or higher, and to 50 mass%, preferably 45 mass% or less, and more preferably 40 mass% or less based on the total mass of the copolymer, in view of improved deposition of polyacrylate microcapsules onto hair.

The vinyl monomer (A) may be contained at a level of from 10 mass% to 50 mass% based on the total mass of the copolymer, preferably at a level of from 15 mass% to 45 mass% based on the total mass of the copolymer, more preferably at a level of from 20 mass% to 40 mass% based on the total mass of the copolymer, more preferably at a level of from 25 mass% to 40 mass% based on the total mass of the copolymer.

Non-limited example of the vinyl monomer (A) having a carboxyl group include, for example, unsaturated carboxylic acid monomers having a carbon number of 3 to 22. The unsaturated carboxylic acid monomer may have preferably a carbon number of 4 or more, still preferably a carbon number of 20 or less, more preferably a carbon number of 18 or less, still more preferably a carbon number of 10 or less, and even more preferably a carbon number of 6 or less. Furthermore, the number of carboxyl groups in the vinyl monomer (A) may be preferably from 1 to 4, more preferably from 1 to 3, even more preferably from 1 to 2, and most preferably 1.

In view of improved deposition of cationic surfactants, fatty compounds and/or silicones, the vinyl monomer (A) may be preferably an unsaturated carboxylic acid monomer expressed by the following formula (2) or formula (3), more preferably those expressed by the formula (2)

$$CH_2=C(R^3)-CO-(O-(CH_2)_m-CO)_n-OH \qquad (2)$$

wherein: $R^3$ represents a hydrogen atom or a methyl group; m represents an integer of 1 to 4, preferably of 2 to 3; and n represents an integer of 0 to 4, preferably of 0 to 2, and most preferably of 0;

$$CH_2=C(R^4)-COO-(CH_2)p-OOC-(CH_2)q-COOH \qquad (3)$$

wherein: $R^4$ represents a hydrogen atom or a methyl group; p and q independently represent an integer of 2 to 6, preferably of 2 to 3.

The vinyl monomer (A) which is preferably an unsaturated carboxylic acid monomer expressed by the following formula (2) may include (meth)acrylic acid, crotonic acid, maleic acid, fumaric acid, itaconic acid, angelic acid, tiglic acid, acrylic acid 2-carboxy ethyl acrylate oligomer, and the like. Among them, preferred are acrylic acid and methacrylic acid, and more preferred is acrylic acid.

The vinyl monomer (A) which is preferably an unsaturated carboxylic acid monomer expressed by the following formula (3) may include acryloyloxyethyl succinate, 2-methacryloyloxyethyl succinate, and the like.

Vinyl Monomer (B)

The copolymer contains a vinyl monomer (B). The copolymer may contain one type of the vinyl monomer (B), or may contain two or more types of the vinyl monomer (B). The vinyl monomer (B) is preferably nonionic.

The vinyl monomer (B) may be contained at a level of from 50 mass% based on the total mass of the copolymer, and to 90 mass% based on the total mass of the copolymer, preferably to 85 mass%, more preferably to 80 mass%, still more preferably 75 mass% based on the total mass of the copolymer, in view of improved deposition of polyacrylate microcapsules onto hair.

The vinyl monomer (B) may be contained at a level of from 50 mass% to 90 mass% based on the total mass of the copolymer, preferably at a level of from 55 mass% to 85 mass% based on the total mass of the copolymer, more preferably at a level of from 60 mass% to 80 mass% based on the total mass of the copolymer, even more preferably at a level of from 60 mass% to 75 mass% based on the total mass of the copolymer.

The vinyl monomers (B) useful herein are those expressed by formula (1)

$$CH_2=C(R^1)-CO-X-(Q-O)_r-R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an

oxygen atom or an NH group; and in the structure $-(Q-O)_r-R^2$, the number of atoms bonded in a straight chain, i.e. the number of atoms bonded linearly to each other in the structure of $-(Q-O)_r-R^2$, is 70 or less.

In the formula (1), $R^1$ may be a hydrogen atom or a methyl group.

$R^2$ represents a hydrogen atom or an alkyl group having a carbon number of 1 to 5, each of which may have a substitution group. If $R^2$ has a substitution group, the substitution group is a substitution group that does not react with other parts of the copolymer. The vinyl monomer (B) may be preferably hydrophilic, and therefore $R^2$ may be preferably a hydrogen atom or an alkyl group having a carbon number of 1 to 3, and more preferably a hydrogen atom or an alkyl group having a carbon number of 1 or 2.

X represents an oxygen atom or an NH group. X may represent preferably an oxygen atom.

Q represents an alkylene group having a carbon number of 2 to 4, which may also have a substitution group. Q may represent preferably an alkylene group having a carbon number of 2 to 3, which may also have a substitution group, and more preferably an alkylene group having a carbon number of 2 to 3 without any substitution group. If the alkylene group of Q has a substitution group, it is preferred that such substitution group does not react with other parts of the copolymer, more preferably such substitution group has a molecular weight of 50 or less. Still more preferably such substitution group may have a molecular weight that is smaller than the one of the structural moiety of -(Q-O)r-. The substitution group of the alkylene group of Q may be preferably selected from the group consisting of a hydroxyl group, a methoxy group, and an ethoxy group.

r represents an integer of 2 to 15. r may represent preferably an integer of 3 or higher, and preferably 12 or less, in view of improved deposition of polyacrylate microcapsules onto hair.

As described above, in the structure $-(Q-O)r-R^2$, the number of atoms that are bonded by the straight chain, i.e. the number of atoms bonded linearly to each other in the structure of $-(Q-O)_r-R^2$, is 70 or less. For example, if Q represents an n-butylene group, r = 15, and $R^2$ represents an n-pentyl group, the number of atoms that are bonded in the straight chain of the structure $-(Q-O)_r-R^2$ is calculated as 80, which therefore is outside of the scope. The number of atoms bonded in the straight chain in the structure $-(Q-O)_r-R^2$ may be preferably 60 or less, more preferably 40 or less, even more preferably 28 or less, and particularly preferably 20 or less, in view of improved deposition of polyacrylate microcapsules onto hair.

Examples of the vinyl monomer (B) include, methoxy polyethylene glycol (meth)acrylate (where the number of polyethylene glycol repeating units (r in formula (1)) is from 2 to 15), polyethylene glycol (meth)acrylate (where the number of polyethylene glycol repeating units (r in formula (1)) is from 2 to 15), methoxy polyethylene glycol / polypropylene glycol (meth)acrylate (where the number of polyethylene glycol / polypropylene glycol repeating units (r in formula (1)) is from 2 to 15), polyethylene glycol / polypropylene glycol (meth)acrylate (where the number of polyethylene glycol / polypropylene glycol repeating units (r in formula (1)) is from 2 to 15), methoxy polyethylene glycol / polybutylene glycol (meth)acrylate (where the number of polyethylene glycol / polybutylene glycol repeating units (r in formula (1)) is from 2 to 15), polyethylene glycol / polybutylene glycol (meth)acrylate (where the number of polyethylene glycol / polybutylene glycol repeating units (r in formula (1)) is from 2 to 15), methoxy polyethylene glycol (meth)acrylamide (where the number of polyethylene glycol repeating units (r in formula (1)) is from 2 to 15), and polyethylene glycol (meth)acrylamide (where the number of polyethylene glycol repeating units (r in formula (1)) is from 2 to 15).

Among these above, more preferred examples of the vinyl monomer (B) include methoxy polyethylene glycol (meth)acrylate (where the number of polyethylene glycol repeating units (r in formula (1)) is from 3 to 12), and polyethylene glycol (meth)acrylate (where the number of polyethylene glycol repeating units (r in formula (1)) is from 3 to 12).

Vinyl Monomer (B1)

The deposition polymer may further contain a vinyl monomer (B1). The deposition polymer may contain one type of the vinyl monomer (B1), or may contain two or more types of the vinyl monomer (B1). The vinyl monomer (B1) is preferably nonionic.

The deposition polymer may be a terpolymer comprising: a vinyl monomer (A) with a carboxyl group in the structure; a vinyl monomer (B) expressed by the following formula (1):

$$CH_2=C(R^1)-CO-X-(Q-O)_r-R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q-O)_r-R^2$, the number of atoms bonded in a straight chain is 70 or less;

a vinyl monomer (B1) expressed by the following formula (4):

$CH_2=C(R^1)-CO-X-(Q-O)_r-R^2$ (4)

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 50; and X represents an oxygen atom or an NH group; and, in the following structure $-(Q-O)_r-R^2$, the number of atoms bonded in a straight chain is 250 or less;

and wherein the vinyl monomer (A) is contained at a level of from 10 mass% to 40 mass% based on the total mass of the copolymer, the vinyl monomer (B) is contained at level of from 50 mass% to 89 mass% based on the total mass of the copolymer; and

the vinyl monomer (B1) is contained at level of from 1 mass% to 10 mass% based on the total mass of the copolymer.

The vinyl monomer (B1) may be contained at a level of from 1 mass% to 10 mass% based on the total mass of the copolymer, preferably at a level of from 2 mass% to 8 mass% based on the total mass of the copolymer, more preferably at a level of from 3 mass% to 7 mass% based on the total mass of the copolymer.

Hence, the vinyl monomer (A) may be contained at a level of from 10 mass% to 40 mass%, based on the total mass of the copolymer, the vinyl monomer (B) may contained at level of from 50 mass% to 89 mass%, preferably at a level of from 52 mass% to 88 mass%, more preferably at a level of from 55 mass% to 87 mass% based on the total mass of the copolymer; and the vinyl monomer (B1) may contained at level of from 1 mass% to 10 mass%, preferably at a level of from 2 mass% to 8 mass%, more preferably at a level of from 3 mass% to 5 mass% based on the total mass of the copolymer.

The vinyl monomers (B1) useful herein are those expressed by formula (4)

$CH_2=C(R^1)-CO-X-(Q-O)_r-R^2$ (4)

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 50; and X represents an oxygen atom or an NH group; and in the structure $-(Q-O)_r-R^2$, the number of atoms bonded in a straight chain, i.e. the number of atoms bonded linearly to each other in the structure of $-(Q-O)_r-R^2$, is 250 or less.

In the formula (4), $R^1$ may be a hydrogen atom or a methyl group.

$R^2$ represents a hydrogen atom or an alkyl group having a carbon number of 1 to 5, each of which may have a substitution group. If $R^2$ has a substitution group, the substitution group is a substitution group that does not react with other parts of the copolymer. The vinyl monomer (B1) may be preferably hydrophilic, and therefore $R^2$ may be preferably a hydrogen atom or an alkyl group having a carbon number of 1 to 3, and more preferably a hydrogen atom or an alkyl group having a carbon number of 1 or 2.

X represents an oxygen atom or an NH group. X may represent preferably an oxygen atom.

Q represents an alkylene group having a carbon number of 2 to 4, which may also have a substitution group. Q may represent preferably an alkylene group having a carbon number of 2 to 3, which may also have a substitution group, and more preferably an alkylene group having a carbon number of 2 to 3 without any substitution group. If the alkylene group of Q has a substitution group, it is preferred that such substitution group does not react with other parts of the copolymer, more preferably such substitution group has a molecular weight of 50 or less. Still more preferably such substitution group may have a molecular weight that is smaller than the one of the structural moiety of -(Q-O)r-. The substitution group of the alkylene group of Q may be preferably selected from the group consisting of a hydroxyl group, a methoxy group, and an ethoxy group.

r represents an integer of 2 to 50, more preferably of 5 to 40, even more preferably of 10 to 30. r may represent preferably an integer of 10 or higher, and preferably 30 or less, in view of improved deposition of polyacrylate microcapsules onto hair.

As described above, in the structure $-(Q-O)r-R^2$, the number of atoms that are bonded by the straight chain, i.e. the number of atoms bonded linearly to each other in the structure of $-(Q-O)_r-R^2$, is 250 or less. For example, if Q represents an n-butylene group, r = 50, and $R^2$ represents an n-pentyl group, the number of atoms that are bonded in the straight chain of the structure $-(Q-O)r-R^2$ is calculated as 255, which therefore is outside of the scope. The number of atoms bonded in the straight chain in the structure $-(Q-O)r-R^2$ may be preferably 240 or less, more preferably 220 or less, even more preferably 210 or less, and particularly preferably 200 or less, in view of improved deposition of polyacrylate microcapsules onto hair.

Examples of the vinyl monomer (B1) include, methoxy polyethylene glycol (meth)acrylate (where the number of polyethylene glycol repeating units (r in formula (4)) is from 2 to 50, preferably from 5 to 40, more preferably from 10 to 30), polyethylene glycol (meth)acrylate (where the number of polyethylene glycol repeating units (r in formula (4)) is from 2 to 50, preferably from 5 to 40, more preferably from 10 to 30), methoxy polyethylene glycol / polypropylene glycol

(meth)acrylate (where the number of polyethylene glycol / polypropylene glycol repeating units (r in formula (4)) is from 2 to 50), polyethylene glycol / polypropylene glycol (meth)acrylate (where the number of polyethylene glycol / polypropylene glycol repeating units (r in formula (4)) is from 2 to 50), methoxy polyethylene glycol / polybutylene glycol (meth)acrylate (where the number of polyethylene glycol / polybutylene glycol repeating units (r in formula (4)) is from 2 to 50), polyethylene glycol / polybutylene glycol (meth)acrylate (where the number of polyethylene glycol / polybutylene glycol repeating units (r in formula (4)) is from 2 to 50), methoxy polyethylene glycol (meth)acrylamide (where the number of polyethylene glycol repeating units (r in formula (4)) is from 2 to 50), and polyethylene glycol (meth)acrylamide (where the number of polyethylene glycol repeating units (r in formula (4)) is from 2 to 50).

Among these above, preferred examples of the vinyl monomer (B1) include (methoxy polyethylene glycol (meth)acrylate (where the number of polyethylene glycol repeating units (r in formula (4)) is from 2 to 50, preferably from 5 to 40, more preferably from 10 to 30), polyethylene glycol (meth)acrylate (where the number of polyethylene glycol repeating units (r in formula (4)) is from 2 to 50, preferably from 5 to 40, more preferably from 10 to 30),

Vinyl Monomer (C)

In addition to the vinyl monomers (A) and (B), the copolymer may further contain a vinyl monomer (C) having an alkyl group having a carbon number of 12 to 22, in view of providing improved deposition of the polyacrylate microcapsules onto hair. When included, the vinyl monomer (c) may be contained at a level of from 40 mass% or less, more preferably 30 mass% or less, even more preferably 25 mass% or less, and still more preferably 20 mass% or less based on the total mass of the copolymer, still even more preferably from 0 to 20 mass% or less based on the total mass of the copolymer, still again even more preferably from 2 to 20 mass% or less based on the total mass of the copolymer.

Preferably, the vinyl monomer (C) may comprise a (meth)acrylate monomer having an alkyl group having a carbon number of 12 to 22, in view of smoothness upon application. Furthermore, vinyl monomers with branched alkyl groups are particularly preferred.

Examples of the (meth)acrylate monomer having an alkyl group having a carbon number of 12 to 22 include myristyl (meth)acrylate, isostearyl (meth)acrylate, stearyl (meth)acrylate, behenyl (meth)acrylate, cetyl (meth)acrylate, lauryl (meth)acrylate, synthetic lauryl (meth)acrylate, (however "synthetic lauryl (meth)acrylate" refers to an alkyl (meth)acrylate wherein the alkyl (meth)acrylate comprises an alkyl group having a carbon number of 12 and an alkyl group having a carbon number of 13 which are mixed), and the like.

The vinyl monomer (C) may comprise more preferably a (meth)acrylate monomer having an alkyl group having a carbon number of 12 to 20, even more preferably a (meth)acrylate monomer having an alkyl group having a carbon number of 16 to 18.

The copolymer may contain only one kind of a constituent unit corresponding to the vinyl monomer (C), or may contain two or more kinds of constituent units corresponding to the vinyl monomer (C).

Other Monomers

In addition to the aforementioned vinyl monomers (A), (B), and (C), the copolymer may also contain other vinyl monomers, as long as the effects of the copolymer are not impaired. Examples of other vinyl monomers include nonionic monomers, amphoteric monomers, semipolar monomers, cationic monomers, as well as monomers containing a polysiloxane group, preferably nonionic monomers with or without polysiloxane group. These other monomers are different from any of the aforementioned vinyl monomers (A), (B), and (C).

Normally the amount of such other monomers, if included, may be 40 mass% or less of the total mass of the copolymer, preferably 30 mass% or less, more preferably 20 mass% or less, and even more preferably 10 mass% or less.

Nonionic monomers may include esters of (meth)acrylic acid and an alcohol having a carbon number of 1 to 22, amides of (meth)acrylic acid and an alkylamine having a carbon number of 1 to 22, monoesters of (meth)acrylic acid and ethylene glycol, 1,3-propylene glycol or the like, as well as esters where the hydroxyl group of the monoester above has been etherified by methanol, ethanol or the like, (meth)acroylmorpholine and the like.

Amphoteric monomers may include (meth)acryl esters having a betaine group, (meth)acrylamide having a betaine group and the like.

Semipolar monomers may include (meth)acrylate esters having an amine oxide group, (meth)acrylamides having an amine oxide group, and the like.

Cationic monomers may include (meth)acrylate esters having a quaternary ammonium group, (meth)acrylamides having a quaternary ammonium group and the like.

The monomer containing a polysiloxane group may be a monomer having a polysiloxane structure and also having a structure that can bond by covalent bonding to the copolymer. The monomer containing a polysiloxane group is a type of constituent unit having relatively high affinity towards silicone oil that is normally used in conjunction in cosmetic material compositions, and are thought to act by bonding the silicone oil to the other component units in the

copolymer and thus increasing the adsorption force of silicone oil to the skin and hair, particularly damaged hair.

The polysiloxane structure may be a structure where two or more repeating structural units expressed by the following formula (5) are linked.

$$-(SiR^5R^6\text{-}O)- \quad (5)$$

In formula (5), $R^5$ and $R^6$ independently represent an alkyl group having a carbon number of 1 to 3 or a phenyl group.

The structure that can be connected to the copolymer via covalent bonding can be a structure that has a vinyl structure such as a (meth)acrylate ester, or (meth)acrylamide and that can copolymerize with other monomer(s), a structure that has a functional group such as a thiol, that can link to the copolymer by chain transfer during polymerization, or a structure that has an isocyanate group, carboxylic acid group, hydroxyl group, amino group, or the like, and that can react and connect with a functional groups on the copolymer, however there is no restriction to these structures.

A plurality of these connectable structures can be present in one monomer containing a polysiloxane group. In the copolymer, the polysiloxane structure may be connected with the main chain by a graft structure, or conversely, the polysiloxane structure may serve as the main chain and another structure may be connected therewith by a graft structure. In addition the polysiloxane structure and another structure may be linearly connected by a block structure.

The monomer containing a polysiloxane group may be preferably expressed by the following formula (6).

$$CH_2=C(R^7)\text{-}Z\text{-}(SiR^8R^9\text{-}O)_s\text{-}R^{10} \quad (6)$$

In the formula (6), $R^7$ represents a hydrogen atom or a methyl group, $R^8$ and $R^9$ independently represent an alkyl group having a carbon number of 1 to 3 or a phenyl group, $R^{10}$ represents an alkyl group having a carbon number of 1 to 8 having a carbon number of, Z represents a divalent linking group or a direct bond, and s represents an integer of 2 to 200.

More preferably, s may be 3 or higher, and even more preferably, s may be 5 or higher, in view of increased affinity to silicone oil. Also, s may be preferably 50 or less, in view of enhanced copolymerization with the other monomers.

Z may represent a divalent linking group or a direct bond, but a linking group containing one or a combination of two or more of the structures suggested below may be preferable. The numbers of structures combined is not particularly restricted, but is usually 5 or less. Furthermore, the direction in which the following structures faces is arbitrary (which end is on the polysiloxane group side). Note, in the following groups, R may represent an alkylene group having a carbon number of 1 to 6 or a phenylene group:

-COO-R-

-CONH-R-

-O-R-

-R-

The monomer containing a polysiloxane group expressed by the aforementioned formula (6), may include, for example, α-(vinyl phenyl) polydimethyl siloxane, α-(vinyl benzyloxy propyl) polydimethyl siloxane, α-(vinyl benzyl) polymethyl phenyl siloxane, α-(methacryloyl oxypropyl) polydimethyl siloxane, α-(methacryloyloxy propyl) polymethyl phenyl siloxane, α-(methacryloyl amino propyl) polydimethyl siloxane and the like. The monomer containing a polysiloxane group can be a single type, or can be two or more types used in combination.

In order to adjust the molecular weight and the viscosity of the copolymer, a cross-linking agent such as a polyfunctional acrylate or the like maybe introduced to the copolymer. However, it is preferred that a cross-linking agent is not included in the copolymer.

## Structure Analysis

The amount of the vinyl monomers (A), (B), and (C) as well as other monomers in the copolymer can be measured using IR absorption or Raman scattering of various functional groups or carbon skeletons, such aa a carbonyl group, amide bond, polysiloxane structure, or by various NMR measurements, e.g. $^1$H-NMR or $^{13}$C-NMR of a methyl group, an amide bond moiety, a methyl or methylene group adjacent thereto, or the like of polydimethylsiloxane.

Weighted Average Molecular Weight

The deposition polymer has a weighted average molecular weight from 3 000 to 2 000 000, preferably from 5 000 to 1 000 000, more preferably from 5 000 to 500 000, even more preferably from 5 000 to 100 000, most preferably from 5 000 to 50 000 as determined by gel permeation chromatography.

The weighted average molecular weight of the deposition polymer may be preferably from 3 000 to 10 000 when the deposition polymer is a copolymer which essentially comprises the vinyl monomer (A) and the vinyl monomer (B). The weighted average molecular weight of the deposition polymer may be preferably from 10 000 to 50 000 when the deposition polymer is a terpolymer which essentially comprises the vinyl monomer (A), the vinyl monomer (B) and the vinyl monomer (B1).

The weighted average molecular weight of the copolymer may be measured by gel permeation chromatography (GPC). The development solvent that is used in gel permeation chromatography is not particularly restricted so long as being a normally used solvent, but for example, the measurement can be performed using a solvent blend of water / methanol / acetic acid / sodium acetate.

**CONDITIONNING AGENT AND CARRIER**

Microcapsules made according to the invention can be employed in compositions which include both a conditioning agent and a carrier. The resulting conditioner compositions may have an oil phase and an aqueous phase. The polyacrylate microcapsules may reside in the aqueous phase of such conditioner compositions.

The conditioner composition comprises from 0.05% to 40 % of a conditioning agent, preferably from 0.5% to 30% of a conditioning agent, more preferably from 2% to 25% of a conditioning agent by weight of the conditioner composition, wherein the conditioning agent is selected from the group consisting of a cationic surfactant, a high melting point fatty compound, a silicone compound, and combinations thereof; and a carrier.

The conditioner composition comprises a total amount of from 0.05% to 40 % of a conditioning agent, preferably from 0.5% to 30% of a conditioning agent, more preferably from 2% to 25% of a conditioning agent by weight of the conditioner composition, wherein the conditioning agent is selected from the group consisting of a cationic surfactant, a high melting point fatty compound, a silicone compound, and combinations thereof; and a carrier.

The conditioner composition may preferably comprise:

(a) from 0.004% to 10% of polyacrylate microcapsules as recited hereinbefore by weight of the conditioner composition;
(b) from 0.05% to 8% of a deposition polymer as recited hereinbefore;
(c) from 0.1% to 20% of a cationic surfactant by weight of the conditioner composition; from 0.1% to 20% of a high melting point fatty compound by weight of the conditioner composition; and optionally from 0.1% to 10% of a silicone compound by weight of the conditioner composition. Alternatively, the conditioner composition may comprise less than 0.1% of a silicone compound by weight of the conditioner composition; and
(d) a carrier.

The conditioner composition may more preferably comprise:

(a) from 0.004% to 10% of polyacrylate microcapsules as recited hereinbefore by weight of the conditioner composition;
(b) from 0.05% to 8% of a deposition polymer as recited hereinbefore;
(c) from 1% to 3.5% of a cationic surfactant by weight of the conditioner composition; from 2% to 10% of a high melting point fatty compound by weight of the conditioner composition; and optionally from 0.1% to 8% of a silicone compound by weight of the conditioner composition; and
(d) a carrier.

The conditioning agent may contain the following components hereinbelow:

A. Cationic Surfactant

The conditioning agent for use in the conditioner composition may contain a cationic surfactant.

The conditioner composition may comprise from 0.05% to 20% of a cationic surfactant, preferably from 0.1% to 8% of a cationic surfactant, more preferably from 0.5% to 5% of a cationic surfactant, even more preferably from 1% to 3.5% of a cationic surfactant by weight of the conditioner composition.

The cationic surfactant may be included in the conditioner composition such that the mole % of the cationic

surfactant to a sum of the cationic surfactant and the high melting point fatty compound is from 10% to 60%, preferably from 15% to 50%, more preferably from 20% to 35%. If the mole% is too low, the composition may provide inferior wet friction. If the mole% is too high, the composition may provide an inferior product texture.

A variety of cationic surfactants including mono- and di-alkyl chain cationic surfactants can be used in the conditioner composition. Mono-alkyl chain cationic surfactants may be used in order to provide a consumer desired gel matrix and wet conditioning benefits. Such mono-alkyl cationic surfactants may include, for example, mono-alkyl quaternary ammonium salts and mono-alkyl amines.

Cationic surfactants such as di-alkyl chain cationic surfactants may be used in combination with mono-alkyl chain cationic surfactants. Such di-alkyl chain cationic surfactants may include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

Cationic surfactants useful herein may also include, for example, mono-alkyl quaternized ammonium salt cationic surfactant having one long alkyl chain of from 12 to 30 carbon atoms, mono-alkyl amine cationic surfactant having one long alkyl chain of from 12 to 30 carbon atoms including mono-alkyl amidoamine cationic surfactant. Mono-alkyl quaternized ammonium salt cationic surfactants are preferred. Additionally, cationic surfactants may include di-alkyl quaternized ammonium salt cationic surfactant having two long alkyl chain of from 12 to 30 carbon atoms which may be used together with the above mono-alkyl cationic surfactants.

Mono-alkyl quaternized ammonium salt cationic surfactant

The conditioner composition may preferably comprise a mono-alkyl quaternized ammonium salt cationic surfactant. The mono-alkyl quaternized ammonium salt cationic surfactant may be included in the composition at a level of from 0.1% to 8%, preferably from 0.2% to 6%, more preferably from 0.5% to 5% by weight of the conditioner composition.

The mono-alkyl quaternized ammonium salt cationic surfactant may be included such that the mole % of the mono-alkyl quaternized ammonium salt cationic surfactant to a sum of the mono-alkyl quaternized ammonium salt cationic surfactant and the high melting point fatty compound is from 10% to 60%, preferably from 15% to 50%, more preferably from 20% to 35%. If the mole% is too low, the compositions may tend to provide increased wet friction. If the mole% is too high, the composition may provide an inferior product texture.

The mono-alkyl quaternized ammonium salt cationic surfactants useful herein may be those having one long alkyl chain of preferably from 12 to 30 carbon atoms, more preferably from 16 to 24 carbon atoms, still more preferably from 18 to 22 carbon atoms, even more preferably 22 carbon atoms, in view of conditioning benefits. Such mono-alkyl quaternized ammonium salt cationic surfactants useful herein are, for example, those having the formula (I):

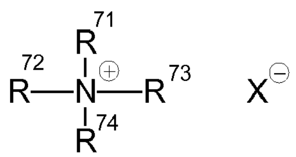

(I)

wherein one of $R^{71}$, $R^{72}$, $R^{73}$ and $R^{74}$ is selected from an aliphatic group of from 12 to 30 carbon atoms, more preferably from 16 to 24 carbon atoms, still more preferably from 18 to 22 carbon atoms, even more preferably 22 carbon atoms or an aromatic group, or an alkoxy group having up to 30 carbon atoms, a polyoxyalkylene wherein the alkylene group has up to 30 carbon atoms, an alkylamido group wherein the alkyl group has up to 30 carbon atoms, a hydroxyalkyl group wherein the alkyl group has up to 30 carbon atoms, an aryl group or an alkylaryl group wherein the alkyl group has up to 30 carbon atoms; the remainder of $R^{71}$, $R^{72}$, $R^{73}$ and $R^{74}$ are independently selected from an aliphatic group of from 1 to 8 carbon atoms, preferably from 1 to 3 carbon atoms or an aromatic group, or an alkoxy group having up to 8 carbon atoms, a polyoxyalkylene wherein the alkylene group has up to 8 carbon atoms, an alkylamido group wherein the alkyl group has up to 8 carbon atoms, a hydroxyalkyl group wherein the alkyl group has up to 8 carbon atoms, an aryl group or an alkylaryl group wherein the alkyl group has up to 8 carbon atoms; and $X^-$ is a salt-forming anion selected from the group consisting of halides such as chloride and bromide, $C_1$-$C_4$ alkyl sulfate which may be methosulfate or ethosulfate, and mixtures thereof. The aliphatic groups can contain, in addition to carbon and hydrogen atoms, ether linkages, and other groups such as amino groups. The longer chain aliphatic groups, e.g., those of 16 carbons, or higher, can be saturated or unsaturated.

Preferably, one of $R^{71}$, $R^{72}$, $R^{73}$ and $R^{74}$ may be selected from an alkyl group of from 12 to 30 carbon atoms, more preferably from 16 to 24 carbon atoms, still more preferably from 18 to 22 carbon atoms, even more preferably 22 carbon atoms; and the remainder of $R^{71}$, $R^{72}$, $R^{73}$ and $R^{74}$ are independently selected from $CH_3$, $C_2H_5$, $C_2H_4OH$, $CH_2C_6H_5$, and mixtures thereof. Such highly preferred cationic surfactants include, for example, behenyl trimethyl ammonium chloride, methyl sulfate or ethyl sulfate.

Such mono-long alkyl quaternized ammonium salts can help to provide an improved slippery feel to wet hair when compared to the slippery feeling produced by multi-long alkyl quaternized ammonium salts. In addition, mono-long alkyl quaternized ammonium salts can provide improved hydrophobicity of the hair and give a smooth feel to dry hair, compared to amine or amine salt cationic surfactants.

Cationic surfactants may be those having a longer alkyl group, i.e., $C_{18}$-$C_{22}$ alkyl group, for example, behenyl trimethyl ammonium chloride, methyl sulfate or ethyl sulfate, and stearyl trimethyl ammonium chloride, methyl sulfate or ethyl sulfate. Cationic surfactants having a longer alkyl group provide reduced irritation to the skin of the consumer compared to cationic surfactants having a shorter alkyl group.

Mono-alkyl amine cationic surfactant

The conditioner composition may contain a mono-alkyl amine cationic surfactant. The mono-alkyl amine cationic surfactant may be included in the composition at a level of from 0.1% to 8%, preferably from 0.2% to 6%, more preferably from 0.5% to 5% by weight of the conditioner composition.

Mono-alkyl amine cationic surfactants useful herein are primary, secondary, and tertiary amines having one long alkyl or alkenyl group of from 12 to 30 carbon atoms, preferably from 16 to 24 carbon atoms, more preferably from 18 to 22 alkyl group. Mono-alkyl amines useful herein also include mono-alkyl amidoamines.

Particularly useful may be tertiary amidoamines having an alkyl group of from 12 to 22 carbon atoms, preferably from 16 to 22 carbon atoms. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines are disclosed in U.S. Patent 4,275,055, Nachtigal, et al.

The above mono-alkyl amine cationic surfactants may be preferably used in combination with acids such as L-glutamic acid, lactic acid, hydrochloric acid, malic acid, succinic acid, acetic acid, fumaric acid, tartaric acid, citric acid, L-glutamic acid hydrochloride, maleic acid, and mixtures thereof; more preferably L-glutamic acid, lactic acid, citric acid. The acid can be used at a molar ratio of the amine to the acid of from 1 : 0.3 to 1 : 2, more preferably from 1 : 0.4 to 1 : 1 in order to improve wet conditioning during and after application of the conditioner composition.

Di-alkyl quaternized ammonium salt cationic surfactants

The conditioner composition may contain a di-alkyl quaternized ammonium salt cationic surfactant. The di-alkyl quaternized ammonium salt cationic surfactant may be included in the composition at a level of from 0.05% to 5%, preferably from 0.1% to 4%, more preferably from 0.2% to 3% by weight of the conditioner composition. When included, it is preferred that the weight ratio of the mono-alkyl cationic surfactant to the di-alkyl quaternized ammonium salt cationic surfactant is from 1:1 to 5:1, more preferably from 1.2:1 to 5:1, still more preferably from 1.5:1 to 4:1, in view of stability in rheology and conditioning benefits.

Di-alkyl quaternized ammonium salt cationic surfactants useful herein may be those having two long alkyl chains of from 12 to 30 carbon atoms, more preferably from 16 to 24 carbon atoms, still more preferably from 18 to 22 carbon atoms. Such di-alkyl quaternized ammonium salts useful herein are those having the formula (I):

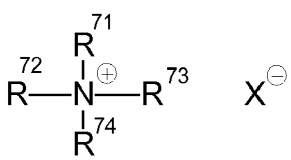

(I)

wherein two of $R^{71}$, $R^{72}$, $R^{73}$ and $R^{74}$ are selected from an aliphatic group of from 12 to 30 carbon atoms, preferably from 16 to 24 carbon atoms, more preferably from 18 to 22 carbon atoms or an aromatic group, or an alkoxy group having up to 30 carbon atoms, a polyoxyalkylene wherein the alkylene group has up to 30 carbon atoms, an alkylamido group wherein the alkyl group has up to 30 carbon atoms, a hydroxyalkyl group wherein the alkyl group has up to 30 carbon atoms, an aryl group or an alkylaryl group wherein the alkyl group has up to 30 carbon atoms; the remainder of $R^{71}$, $R^{72}$, $R^{73}$ and $R^{74}$ are independently selected from an aliphatic group of from 1 to 8 carbon atoms, preferably from 1 to 3 carbon atoms or an aromatic group, or an alkoxy group having up to 8 carbon atoms, a polyoxyalkylene wherein the alkylene group has up to 8 carbon atoms, an alkylamido group wherein the alkyl group has up to 8 carbon atoms,

a hydroxyalkyl group wherein the alkyl group has up to 8 carbon atoms, an aryl group or an alkylaryl group wherein the alkyl group has up to 8 carbon atoms; and $X^-$ is a salt-forming anion selected from the group consisting of halides which may be chloride or bromide, $C_1$-$C_4$ alkyl sulfate which may be methosulfate or ethosulfate, and mixtures thereof. The aliphatic groups can contain, in addition to carbon and hydrogen atoms, ether linkages, and other groups such as amino groups. The longer chain aliphatic groups, e.g., those of 16 carbons, or higher, can be saturated or unsaturated. Preferably, two of $R^{71}$, $R^{72}$, $R^{73}$ and $R^{74}$ are selected from an alkyl group of from 12 to 30 carbon atoms, preferably from 16 to 24 carbon atoms, more preferably from 18 to 22 carbon atoms; and the remainder of $R^{71}$, $R^{72}$, $R^{73}$ and $R^{74}$ are independently selected from $CH_3$, $C_2H_5$, $C_2H_4OH$, $CH_2C_6H_5$, and mixtures thereof.

Such preferred di-alkyl cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

B. High Melting Point Fatty Compound

The conditioner agent for use in the conditioner composition may include a high melting point fatty compound. The high melting point fatty compound as used herein is a fatty compound having a melting point of 25°C or higher, preferably 40°C or higher, more preferably 50°C or higher, in view of stability of the emulsion especially of the gel matrix. Preferably, the high melting point fatty compound as used herein is a fatty compound having a melting point up to 90°C, more preferably up to 80°C, still more preferably up to 65°C, in view of easier manufacturing and easier emulsification. The high melting point fatty compound can be used as a single compound or as a blend or mixture of at least two high melting point fatty compounds. When used as such blend or mixture, the above melting point means the melting point of the blend or mixture.

The high melting point fatty compound may be selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, *e.g.*, some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols may be preferably used. The fatty alcohols useful herein may be those having a carbon number of 14 to 30, or preferably of 16 to 22. These fatty alcohols may be saturated and can be straight or branched chain alcohols. Fatty alcohols may include, for example, cetyl alcohol, stearyl alcohol, behenyl alcohol, and mixtures thereof. More preferred fatty alcohols may be selected from the group consisting of cetyl alcohol, stearyl alcohol and mixtures thereof.

Commercially available high melting point fatty compounds useful herein include: cetyl alcohol, stearyl alcohol, and behenyl alcohol having tradenames KONOL series available from Shin Nihon Rika (Osaka, Japan), and NAA series available from NOF (Tokyo, Japan); pure behenyl alcohol having tradename 1-DOCOSANOL available from WAKO (Osaka, Japan).

High melting point fatty compounds of a single compound of high purity may be used. Single compounds of pure fatty alcohols may be selected from the group consisting of pure cetyl alcohol, stearyl alcohol, and behenyl alcohol. By "pure" herein, what is meant is that the compound has a purity of at least 90%, or even at least 95%. These single compounds of relatively high purity can provide good rinsability from the hair when the consumer rinses off the composition.

The conditioner composition may comprise from 0.1% to 40% of a high melting point fatty compound, preferably from 1% to 20% of a high melting point fatty compound, more preferably from 1.5% to 12% of a high melting point fatty compound, or even more preferably from 2% to 10% of a high melting point fatty compound by weight of the conditioner composition, in view of providing improved conditioning benefits such as slippery feel during the application to wet hair, softness and moisturized feel on dry hair.

C. Nonionic Polymers

The conditioner agent for use in the conditioner composition may include a nonionic polymer. Polyalkylene glycols having a molecular weight of more than 1000 may be useful herein. Useful are those having the following general formula (II):

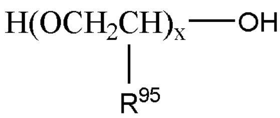

(II)

wherein $R^{95}$ is selected from the group consisting of H, methyl, and mixtures thereof. Polyethylene glycol polymers useful herein are PEG-2M (also known as Polyox WSR® N-10, which is available from Union Carbide and as PEG-2000); PEG-5M (also known as Polyox WSR® N-35 and Polyox WSR® N-80, available from Union Carbide and as PEG-5000 and Polyethylene Glycol 300000); PEG-7M (also known as Polyox WSR® N-750 available from Union Carbide); PEG-9M (also known as Polyox WSR® N-3333 available from Union Carbide); and PEG-14 M (also known as Polyox WSR® N-3000 available from Union Carbide). D. Silicone Compound

The conditioner agent for use in the conditioner composition may include a silicone compound.

It is believed that the silicone compound can provide smoothness and softness on dry hair. The silicone compounds herein can be used at levels by weight of the composition of preferably from 0.1% to 20%, more preferably from 0.5% to 10%, still more preferably from 1% to 8%.

Preferably, the silicone compounds have an average particle size of from 1 micron to 50 microns, in the composition.

The silicone compounds useful herein, as a single compound, as a blend or mixture of at least two silicone compounds, or as a blend or mixture of at least one silicone compound and at least one solvent, have a viscosity of preferably from 1 000 mPa·s to 2 000 000 mPa·s at 25°C.

The viscosity can be measured by means of a glass capillary viscometer as set forth in Dow Corning Corporate Test Method CTM0004, July 20, 1970. Suitable silicone fluids include polyalkyl siloxanes, polyaryl siloxanes, polyalkylaryl siloxanes, polyether siloxane copolymers, amino substituted silicones, quaternized silicones, and mixtures thereof. Other nonvolatile silicone compounds having conditioning properties can also be used.

Preferred polyalkyl siloxanes may include, for example, polydimethylsiloxane, polydiethylsiloxane, and polymethylphenylsiloxane. Polydimethylsiloxane, which is also known as dimethicone, is especially preferred.

The above polyalkylsiloxanes are available, for example, as a mixture with silicone compounds having a lower viscosity. Such mixtures may have a viscosity of preferably from 1 000 mPa·s to 100 000 mPa·s, more preferably from 5 000 mPa·s to 50 000 mPa·s. Such mixtures may preferably comprise: (i) a first silicone having a viscosity of from 100 000 mPa·s to 30 000 000 mPa·s at 25°C, preferably from 100 000 mPa·s to 20 000 000 mPa·s; and (ii) a second silicone having a viscosity of from 5 mPa·s to 10 000 mPa·s at 25°C, preferably from 5 mPa·s to 5 000 mPa·s. Such mixtures useful herein may include, for example, a blend of dimethicone having a viscosity of 18 000 000 mPa·s and dimethicone having a viscosity of 200 mPa·s available from GE Toshiba, and a blend of dimethicone having a viscosity of 18 000 000 mPa·s and cyclopentasiloxane available from GE Toshiba.

The silicone compounds useful herein may also include a silicone gum. The term "silicone gum", as used herein, means a polyorganosiloxane material having a viscosity at 25°C of greater than or equal to 1 000 000 $mm^2/s$ (1 000 000 centistokes). It is recognized that the silicone gums described herein can also have some overlap with the above-disclosed silicone compounds. This overlap is not intended as a limitation on any of these materials. The "silicone gums" will typically have a mass molecular weight in excess of 200 000, generally between 200 000 and 1 000 000. Specific examples may include polydimethylsiloxane, poly(dimethylsiloxane methylvinylsiloxane) copolymer, poly(dimethylsiloxane diphenylsiloxane methylvinylsiloxane) copolymer and mixtures thereof. The silicone gums are available, for example, as a mixture with silicone compounds having a lower viscosity. Such mixtures useful herein include, for example, Gum/Cyclomethicone blend available from Shin-Etsu.

Silicone compounds useful herein may also include amino substituted materials as disclosed in European patent application EP17171644.2 from p. 32, 1. 14 to p. 33, 1. 17 as filed, which is hereby incorporated by reference. Preferred aminosilicones may include, for example, those which conform to the general formula (I):

$$(R_1)_aG_{3-a}\text{-Si-(-OSiG}_2)_n\text{-(-OSiG}_b(R_1)_{2-b})_m\text{-O-SiG}_{3-a}(R_1)_a$$

wherein G is hydrogen, phenyl, hydroxy, or $C_1$-$C_8$ alkyl, preferably methyl; a is 0 or an integer having a value from 1 to 3, preferably 1; b is 0, 1 or 2, preferably 1; n is a number from 0 to 1999; m is an integer from 0 to 1999; the sum of n and m is a number from 1 to 2000; a and m are not both 0; $R_1$ is a monovalent radical conforming to the general formula $CqH_{2q}L$, wherein q is an integer having a value from 2 to 8 and L is selected from the following groups: $-N(R_2)CH_2-CH_2-N(R_2)_2$; $-N(R_2)_2$; $-N(R_2)_3A$ ; $-N(R_2)CH_2-CH_2-NR_2H_2A$ ; wherein $R_2$ is hydrogen, phenyl, benzyl, or a saturated hydrocarbon radical, preferably an alkyl radical from $C_1$ to $C_{20}$; A is a halide ion.

Silicone Polymer Containing Quaternary Groups

Silicone compounds useful herein may include, for example, a Silicone Polymer Containing Quaternary Groups comprising terminal ester groups, having a viscosity up to 100000 mPa·s and a D block length of greater than 200 D units, as disclosed in European patent application EP17171644.2 from p. 33, 1. 19 to p. 38, 1. 28 as filed, which is hereby incorporated by reference.

E. Gel Matrix

The above cationic surfactants, together with high melting point fatty compounds and an aqueous carrier, may form a gel matrix in the conditioner composition.

The gel matrix is suitable for providing various conditioning benefits such as slippery feel during the application to wet hair and softness and moisturized feel on dry hair.

Preferably, especially when the gel matrix is formed, the total amount of the cationic surfactant and the high melting point fatty compound may be from 4.5%, preferably from 5.0%, more preferably from 5.5% by weight of the conditioner composition, in view of providing the desired benefits, and to 15%, preferably to 14%, more preferably to 13%, still more preferably to 10% by weight of the conditioner composition, in view of spreadability and product appearance. Furthermore, when the gel matrix is formed, the cationic surfactant and the high melting point fatty compound may be contained at a level such that the weight ratio of the cationic surfactant to the high melting point fatty compound is in the range of, preferably from 1:1 to 1:10, more preferably from 1:1.5 to 1:7, still more preferably from 1:2 to 1:6, in view of providing improved wet conditioning benefits.

Carrier

The formulations disclosed herein can be in the form of pourable liquids (under ambient conditions). Such compositions will therefore typically comprise a carrier, which is present at a level of from 20% to 95%, preferably from 60% to 85% by weight of the conditioner composition.

The conditioner composition may comprise an aqueous carrier. The carrier may comprise water, or a miscible mixture of water and organic solvent. The carrier may also comprise water with minimal or no significant concentrations of organic solvent, except as otherwise incidentally incorporated into the composition as minor ingredients of other essential or optional components. Preferably, the aqueous carrier may essentially comprise water. Deionized water may be preferably used. Water from natural sources including mineral cations can also be used, depending on the desired characteristic of the product. Generally, the conditioner composition may comprise from 20% to 99%, preferably from 30% to 95%, and more preferably from 80% to 90% water by weight of the conditioner composition.

The carrier may comprise water and water solutions of lower alkyl alcohols and polyhydric alcohols. The lower alkyl alcohols useful herein are monohydric alcohols having a carbon number of 1 to 6, preferably of 2 to 3. The lower alkyl alcohols may be ethanol and/or isopropanol. The polyhydric alcohols useful herein may include propylene glycol, hexylene glycol, glycerin, and propane diol.

ADDITIONAL COMPONENTS

The conditioner composition may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from 0.001% to 10%, preferably up to 5% by weight of the conditioner composition.

A wide variety of other additional components can be formulated into the present compositions. These include: other conditioning agents such as hydrolysed collagen with tradename Peptein 2000 available from Hormel, vitamin E with tradename Emix-d available from Eisai, panthenol available from Roche, panthenyl ethyl ether available from Roche, hydrolysed keratin, proteins, plant extracts, and nutrients; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; coloring agents, such as any of the FD&C or D&C dyes; perfumes; ultraviolet and infrared screening and absorbing agents such as benzophenones; and antidandruff agents such as zinc pyrithione.

Method of Making Conditioner Formulations

The conditioner composition can be prepared by any conventional method well known in the art.

**Method of Manufacture**

The conditioner compositions can be prepared by the process comprising, preferably in that order, the steps of: a) adding a deposition polymer to a conditioning agent to form a pre-conditioner composition, wherein the deposition polymer is a copolymer comprising: a vinyl monomer (A) with a carboxyl group in the structure; and a vinyl monomer (B) expressed by the following formula (1):

$$CH_2=C(R^1)\text{-}CO\text{-}X\text{-}(Q\text{-}O)_r\text{-}R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q\text{-}O)_r\text{-}R^2$, the number of atoms bonded in a straight chain is 70 or less; and wherein the vinyl monomer (A) is contained at a level of from 10 mass% to 50 mass% based on the total mass of the copolymer, and the vinyl monomer (B) is contained at level of from 50 mass% to 90 mass% based on the total mass of the copolymer, wherein the conditioning agent is selected from the group consisting of a cationic surfactant, a high melting point fatty compound, a silicone compound, and combinations thereof; and a carrier; and b) adding polyacrylate microcapsules, wherein a block co-polymer is disposed on an outer surface of the polyacrylate microcapsules, and wherein said block co-polymer has the formula and is defined as set out hereinbefore; to the resulting pre-conditioner composition of step (a).

Alternatively, the conditioner compositions can be prepared by the process, preferably in that order, the steps of: a) adding polyacrylate microcapsules, wherein a block co-polymer is disposed on an outer surface of the polyacrylate microcapsules, and wherein said block co-polymer has the formula and is defined as set out hereinbefore; to a conditioning agent selected from the group consisting of a cationic surfactant, a high melting point fatty compound, a silicone compound, and combinations thereof; and a carrier, to form a pre-conditioner composition; and b) adding a deposition polymer to the resulting pre-conditioner composition of step (a), wherein the deposition polymer is a copolymer comprising: a vinyl monomer (A) with a carboxyl group in the structure; and a vinyl monomer (B) expressed by the following formula (1):

$$CH_2=C(R^1)\text{-}CO\text{-}X\text{-}(Q\text{-}O)_r\text{-}R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q\text{-}O)_r\text{-}R^2$, the number of atoms bonded in a straight chain is 70 or less; and wherein the vinyl monomer (A) is contained at a level of from 10 mass% to 50 mass% based on the total mass of the copolymer, and the vinyl monomer (B) is contained at level of from 50 mass% to 90 mass% based on the total mass of the copolymer.

Alternatively, the conditioner compositions can be prepared by the process comprising, preferably in that order, the steps of: 1) combining polyacrylate microcapsules, wherein a block co-polymer is disposed on an outer surface of the polyacrylate microcapsules, and wherein said block co-polymer has the formula and is defined as set out hereinbefore; with a deposition polymer which is which is a copolymer comprising: a vinyl monomer (A) with a carboxyl group in the structure; and a vinyl monomer (B) expressed by the following formula (1):

$$CH_2=C(R^1)\text{-}CO\text{-}X\text{-}(Q\text{-}O)_r\text{-}R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q\text{-}O)_r\text{-}R^2$, the number of atoms bonded in a straight chain is 70 or less; and wherein the vinyl monomer (A) is contained at a level of from 10 mass% to 50 mass% based on the total mass of the copolymer, and the vinyl monomer (B) is contained at level of from 50 mass% to 90 mass% based on the total mass of the copolymer, to form a premix; and 2) adding the premix to a conditioner agent as set out hereinbefore and a carrier.

The deposition polymer may be added to the polyacrylate microcapsules or the conditioning agent by mixing the deposition polymer with the polyacrylate microcapsules or the conditioning agent using a conventional mixing device, such as a spatula, in a conventional mixing container, such as a glass jar. On a commercial scale, the deposition polymer can be added to the polyacrylate microcapsules or the conditioning agent via conventional, commercial-scale mixing equipment.

The resulting mixture can be then prepared when the polyacrylate microcapsules are in one or more forms, including slurry form, neat particle form, and spray dried particle form. The polyacrylate microcapsules may be also combined with the conditioning agent by methods that include mixing and/or spraying.
The polyacrylate microcapsules can be formulated into any suitable form and prepared by any process chosen by the formulator, non-limiting examples of which are described in U.S. 5,879,584; U.S. 5,691,297; U.S. 5,574,005; U.S. 5,569,645; U.S. 5,565,422; U.S. 5,516,448; U.S. 5,489,392; U.S. 5,486,303.

**Product Forms and Use**

The conditioner compositions can be in the form of rinse-off products or leave-on products, and can be formulated in a wide variety of product forms, including but not limited to creams, gels, emulsions, mousses and sprays.
The conditioner composition is especially suitable for rinse-off hair conditioner. Such compositions are preferably used by following steps:

(i) after shampooing hair, applying to the hair an effective amount of the conditioner compositions for conditioning the hair; and
(ii) then rinsing the hair.

Alternatively, the conditioner composition may be in the form of a leave-on conditioner composition. The term "leave-on" as used herein means that the conditioner composition is intended to be applied to and allowed to remain on the keratinous tissue, preferably without rinsing the conditioner composition out of the hair. A leave-on conditioner composition is a form that is to be distinguished from compositions, which are applied to the hair and subsequently (in a few minutes or less) removed either by washing, rinsing, wiping, or the like.
Leave-on conditioner compositions exclude rinse-off applications such as shampoos, rinse-off conditioners, facial cleansers, hand cleansers, body wash, or body cleansers.
The leave-on conditioner composition may be left on the keratinous tissue for at least 15 minutes.
For a leave-on conditioner composition, the conditioner composition may be applied to wet or damp hair prior to drying of the hair. After such conditioner composition is applied to the hair, the hair may be dried and styled in accordance with the preference of the user. Alternatively, the conditioner composition may be applied to already dry hair, and the hair may be then combed or styled, and dried in accordance with the preference of the user.
An aspect of the present invention is related to the use of a deposition polymer in a conditioner composition comprising polyacrylate microcapsules, wherein a block co-polymer is disposed on an outer surface of the polyacrylate microcapsules, and wherein said block co-polymer has a formula:

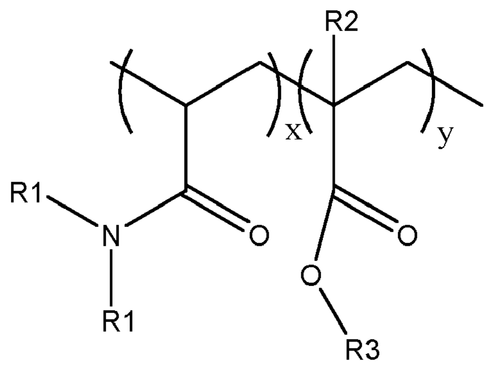

wherein

x and y are integers independently selected such that a molar ratio of monomer units represented by x to monomer units represented by y is from 1.6:1 to 2.5:1;
each R1 is independently selected from the group consisting of H and $CH_3$;
each R2 is independently selected from the group consisting of H and $CH_3$; and
each R3 is independently a $C_1$-$C_{18}$ alkyl group;

wherein said block co-polymer has a number average molecular weight of at least 11 kDa according to the Molecular Weight Test Method as disclosed herein; a conditioning agent and a carrier. The deposition polymer may be used in the range from 0.05% to 8% by total weight of the conditioner composition. The deposition polymer comprises a copolymer

comprising: a vinyl monomer (A) with a carboxyl group in the structure; and a vinyl monomer (B) expressed by the following formula (1):

$$CH_2=C(R^1)\text{-}CO\text{-}X\text{-}(Q\text{-}O)_r\text{-}R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q\text{-}O)_r\text{-}R^2$, the number of atoms bonded in a straight chain is 70 or less; and wherein the vinyl monomer (A) is contained at a level of from 10 mass% to 50 mass% based on the total mass of the copolymer, and the vinyl monomer (B) is contained at level of from 50 mass% to 90 mass% based on the total mass of the copolymer.

The deposition polymer is used in a conditioner composition comprising polyacrylate microcapsules, wherein a block co-polymer is disposed on an outer surface of the polyacrylate microcapsules, and wherein said block co-polymer has the formula and is defined as set out hereinbefore, a conditioning agent and a carrier for increasing the deposition of the polyacrylate microcapsules onto hair for a period of at least 4 hours, preferably from 4 to 24 hours.

Alternatively, the deposition polymer is used in a conditioner composition comprising polyacrylate microcapsules, wherein a block co-polymer is disposed on an outer surface of the polyacrylate microcapsules, and wherein said block co-polymer has the formula and is defined as set out hereinbefore, a conditioning agent and a carrier for providing a relatively long-lasting odor benefit for a period of at least 4 hours, preferably for a period of at least 24 hours, more preferably from 4 to 24 hours.

## TEST METHODS

It is understood that the test methods that are disclosed in the Test Methods Section of the present application should be used to determine the respective values of the parameters of Applicants' invention as such invention is described and claimed herein.

### MOLECULAR WEIGHT TEST METHOD

The following test method is used to determine the number average molecular weight of the block co-polymer.

The number average molecular weights ("Mn") and weight average molecular weights ("Mw") and polydispersity index ("PDI") of the block co-polymer are determined using gel permeation chromatography ("GPC") with an Agilent 1100 Series HPLC equipped with a PSS SDV Lux column (5 $\mu$m) guard column and two PSS SDV Linear XL Lux Columns (5 $\mu$m) (linear range of MW = 100 - $3 \times 10^6$ g/mol), using filtered tetrahydrofuran ("THF") containing 200 ppm 2,6-bis(1,1-dimethylethyl)-4-methylphenol ("BHT") mobile phase at a flow rate of 1.0 mL/min at ambient temperature and miniDAWN TREOS light scattering (60 mW GaAs linearly polarized laser, 658 nm), Optilab rEX differential refractometer (light source = 658 nm; Wyatt Technology Corporation) and ViscoStar-II viscometer (Model NO: WV2-03, Wyatt Technology Corporation) detectors.

Samples for analysis are prepared at a known concentration in the range of 1 to 5 mg/mL. The block co-polymer is dissolved in eluent THF and filtered through 0.2 $\mu$m membrane filters before injection. ASTRA software v. 5.4.14 is used to determine the molecular weight averages and polydispersity. Number average molecular weight and polydispersity index are calculated and reported for the block co-polymer.

### WATER UPTAKE VALUE ("WUV") TEST METHOD

The following test method is used to determine the Water Uptake Value ("WUV") of the block co-polymer.

Polymer test materials are analyzed to determine their capacity to take up or absorb water via the water uptake test method herein. This water uptake adsorption capacity is determined by measuring the weight (in grams) of water uptake per gram of dry polymer test material.

Opened-ended, heat-sealable, empty teabag bags are used to contain samples of the test polymer during exposure to water. These empty teabag bags are made from oxygen-bleached filter paper comprising thermoplastic fibers, abaca fibers, and cellulosic fibers, and have bag dimensions of approximately 5.7 cm x 6.4 cm (such as those available from the Special Tea Company, Orlando, Florida, U.S.A.. Web: www.specialteacompany.com). Ten empty and dry teabag bags are immersed for 24 hours in hard water having a pH of 7, a calcium carbonate hardness of 154 mg/L, and a temperature between 21°C and 25°C. After the immersion, the empty tea bags are removed from the water and placed on a dry paper towels for 15 seconds to remove excess moisture via blotting. Each of the 10 empty wet bags is weighed individually with an accuracy of $\pm$ 0.1 mg and the individual weight results are recorded. These weight data

values are averaged to determine the average Empty Wet Bag weight.

A mass of between 300 mg and 600 mg of the dry polymer material being tested is weighed into each of ten dry and labelled open-ended teabags. The weight of each of the ten replicate dry polymer test samples is recorded as an Initial Dry Polymer sample weight, and the open edges of the bags are then heat-sealed to secure the polymer sample inside each bag. Each of the ten polymer-filled bags are then immersed for 24 hours in hard water having a pH of 7, a calcium carbonate hardness of 154 mg/L, and a temperature between 21°C and 25°C. After the immersion, the bags are removed from the water and placed on a dry paper towel for 15 seconds to remove excess moisture via blotting. Each filled, wet bag is then weighed individually with an accuracy of 0.1 mg and the results are recorded as the individual Filled Wet Bag weights.

The average Empty Wet Bag weight is subtracted from each individual Filled Wet Bag weight to calculate the individual Wet Polymer weight for each of the ten samples. For each of the ten samples, the individual weight of Water Taken Up is calculated by subtracting the Initial Dry Polymer sample weight from the Wet Polymer weight, for each sample respectively. Water Uptake per Gram of Dry Polymer is calculated for each of the ten replicate samples, by dividing the individual weight of Water Taken Up by the individual weight of Initial Dry Polymer, for each respective sample, in accordance with the following three equations:

Filled Wet Bag (g) - average Empty Wet Bag (g) = Wet Polymer (g)
Wet Polymer (g) - Initial Dry Polymer (g) = Water Taken Up (g)
Water Taken Up (g) / Initial Dry Polymer (g) = Water Uptake per Gram of Dry Polymer (g/g)

The Water Uptake Values of the sample polymer are calculated from the ten replicate samples and then averaged. This average result is the value that is reported as the Water Uptake Value in grams of water per gram of dry polymer (in units of grams per gram), for the polymer material being tested.

VISCOSITY OF THE CONDITIONER COMPOSITION

Viscosity of liquid finished product is measured using an AR 550 rheometer / viscometer from TA instruments (New Castle, DE, USA), using parallel steel plates of 40 mm diameter and a gap size of 500 μm. The high shear viscosity at 20 $s^{-1}$ and low shear viscosity at 0.05 $s^{-1}$ is obtained from a logarithmic shear rate sweep from 0.1 $s^{-1}$ to 25 $s^{-1}$ in 3 min time at 21 °C.

VOLUME WEIGHTED MEDIAN PARTICLE SIZE

The volume weighted median particle size of the polyacrylate microcapsules is measured using an Accusizer 780A, made by Particle Sizing Systems, Santa Barbara CA. The instrument is calibrated from 0 to 300 μm using Duke particle size standards. Samples for particle size evaluation are prepared by diluting 1 g emulsion, if the volume weighted median particle size of the emulsion is to be determined, or 1 g of capsule slurry, if the finished capsule volume weighted median particle size is to be determined, in 5 g of de-ionized water and further diluting 1 g of this solution in 25 g of water.

1 g of the most dilute sample is added to the Accusizer and the testing initiated, using the autodilution feature. The Accusizer should be reading in excess of 9200 counts/second. If the counts are less than 9200 additional sample should be added. The Accusizer will dilute the test sample until 9200 counts/second and initiate the evaluation. After 2 min of testing the Accusizer will display the results, including volume-weighted median size.

The broadness index can be calculated by determining the particle size at which 95% of the cumulative particle volume is exceeded (95% size), the particle size at which 5% of the cumulative particle volume is exceeded (5% size), and the median volume-weighted particle size (50% size-50% of the particle volume both above and below this size).

$$\text{Broadness Index (5)} = ((95\%\ \text{size})-(5\%\ \text{size})/50\%\ \text{size}).$$

DETERMINATION OF HYDROLYSIS DEGREE

The hydrolysis degree, defined as percent hydrolysis means mole % hydrolysis of polyvinyl alcohol determined as follows. This measurement is a measure of the number of acetate groups that are replaced by hydroxyl groups during alcoholysis. A saponification number is also determined directly proportional to the percent hydrolysis.

Weigh a sample on an analytical balance and record the weight. Transfer the weighed material into a 500 mL flask and place a magnetic stir bar in the flask.

Add 200 mL of methanol/water solution to the flask. Methanol/distilled water solution, 25% methanol, 75% distilled water v/v, prepared by adding reagent grade methanol to distilled water. Place the flask on a magnetic stirrer

and slurry for 5 to 10 min. Add 5 drops of phenolphthalein indicator solution to the flask. The indicator solution is 1% Phenolphthalein in an ethanol water solution (w/v). The water ethanol solution comprises 50% ethanol and 50% distilled water.

Add 10 mL of 0.5N NaOH to the flask. Heat the contents of the flask to boiling and reflux for 30 min minimum and until the polyvinyl alcohol is completely dissolved. Rinse the condenser walls with 20-30 mL of distilled water. Cool the flask to room temperature. Zero the burette, and titrate the solution to a colorless endpoint. Record the titration volumes in mL.

Net volume of HCl titration in mL = (blank HCl titration in mL) - (sample HCl titration in mL)

$$\text{Hydrolysis (mole \%)} = \left(1 - \frac{(44) \times (S)}{(56100 - (42 \times S))}\right) \times 100$$

$$\text{Saponification Number} = \frac{(\text{net volume of HCl titration in mL}) \times (\text{N HCl} \times (56.1)}{(\text{sample wt}) \times (\text{Weight \% solids expressed as a decimal})}$$

where:

S = Saponification Number,
N HCl = Normality of hydrochloric acid used

DETERMINATION OF VISCOSITY OF THE POLYVINYL ALCOHOL SHELL MATERIAL

Viscosity is measured using a Brookfield LV series viscometer or equivalent, measured at 4.00% +/- 0.05% solids.

Weigh a 500 mL beaker and stirrer. Record the weight. Add 16.00 + 0.01 g of a polyvinyl alcohol (PVOH) sample to the beaker. Add approximately 350-375 mL of deionized water to the beaker and stir the solution. Place the beaker into a hot water bath with the cover plate. Agitate at moderate speed for 45 min to 1 hour, or until the PVOH is completely dissolved. Turn off the stirrer. Cool the beaker to approximately 20°C.

Calculate the final weight of the beaker as follows:

$$\text{Final weight} = (\text{weight of empty beaker \& stirrer}) + (\text{\% solids as decimal} \times 400)$$

Example: weight of empty beaker & stirrer = 125.0 g
% solids of PVOH = 97.50% or 0.9750 as decimal Final weight = 125.0 + (0.9750 x 400) = 515.0 g

Zero the top loading balance and place the beaker of PVOH solution with a propeller on it. Add deionized water to bring the weight up to the calculated final weight.

Dispense the sample of 4% PVOH solution into the chamber of the viscometer, insert the spindle and attach it to the viscometer. Sample adapter (SSA) with chamber SC4-13RPY, Ultralow adapter. The spindles are SC4-18 and 00. Allow the sample to achieve equilibration at 20°C temperature. Start the viscometer and record the steady state viscosity value.

Determine solids content.

Weight % solids expressed as a decimal =
Solids content of the sample should be 4.00 + 0.05% to calculate viscosity.

Report viscosity <13 cP to nearest 0.01 cP, 13-100 cP to nearest 0.1 cP; viscosities over 100 cP are reported to the nearest 1 cP.

Corrections to the measured viscosity are not necessary if the calculated solution solids is 4.00%. Otherwise, use the following equation to correct the measured viscosity for solution solids deviations.

$$\text{Log}_e \text{ Corrected Viscosity} = \frac{(\text{Log}_e \text{ Measured Viscosity})}{(\text{percent solids}) \times (0.2060) + (0.1759)}$$

$$\text{Corrected Viscosity} = 2.718282(\text{Log Corrected Viscosity})$$

POLYVINYL ALCOHOL AVERAGE NUMBER MOLECULAR WEIGHT

The following test method is used to determine the average number molecular weight of the polyvinyl alcohol of the shell material. A weight % of PVOH in water solution is prepared and the sample is injected into a GPC instrument:

Malvern Viscotek GPCmax VE 2001 sample module connected to a Malvern Viscotek Model 305 TDA (Triple Detector Array)
Instrument Settings during analysis:

Solvent: water
Column Set: SOLDEX SB804+802.5
Flow rate: 0.750 mL/min
Injection Volume: 100 μl
Detector Temp: 30°C

DEGREE OF POLYMERIZATION

The following test method is used to determine the degree of polymerization of the polyvinyl alcohol of the shell material. Degree of polymerization is determined from the molecular weight data of the Average Number Molecular Weight test. Using the output from the GPC instrument, Degree of Polymerization is calculated from GPC value for $M_n$

$$\text{Degree of Polymerization}= \frac{Mn}{(86-0.42 \times Degree\ of\ hydrolysis)}$$

DEPOSITION OF MICROCAPSULES ONTO HAIR TEST METHOD

The amount of microcapsules deposited onto hair in a hair conditioning process is evaluated according to the following test method.

Pre-Cleaning of Hair Switches: The water of a stationary shower is preset to a temperature of 37.8°C (100 F) and a flow rate of 57 L (1.5 gallons) per min. 0.1 g of Sodium Lauryl Ether Sulfate per gram of hair switch is applied to the hair switch that has been pre-wet with tap water and lightly squeegeed. The switch is milked for 30 seconds. Then the switch is rinsed with stationary shower rinse for 30 sec, and then squeegeed. The milking and rinsing process are duplicated. The hair switches are air dried overnight.

Application of the conditioner composition to be tested:

In a 50 g first sample jar, 4 g of pre-cleaned of hair switch and 20 g of the conditioner composition to be tested are added. The first sample jar is agitated by hand for 30 seconds to saturate the hair switch with the microcapsule test solution. The hair switch is then removed from the first sample jar and placed into a clean, dry 50 g second sample jar and 20 g of rinse water is added to the second sample jar. The solution remaining in the first sample jar is kept for analysis.

The second sample jar is agitated by hand for 30 sec to rinse the hair switch with the rinse water. The rinse solution is kept in the second sample jar for analysis. The concentrations of microcapsules in the solutions in the first sample jar and second sample jar are analyzed by Horiba DUAL FL-UV-800-C fluorometer. The solutions of the first sample jar and the second sample jar are each transferred to separate testing cuvettes using a plastic transfer pipettes. Each cuvette is placed on the fluorometer and running a 3D EEM plus absorbance scan with the following settings: the starting and ending Excitation Wavelengths were 250 nm and 600 nm, respectively; Excitation Wavelength Increment 3 nm; Emission Coverage Increment: 4.66; CCD Gain: Medium; Integration Time: 0.1 second.

Data processing

Data are analyzed using Aqualog Dual - FL with Origin Software. The process intensity at 318 nm wavelength is selected for data analysis. The amount of microcapsules in each solution are calculated based on calibration curves prepared in the starting tap water solution or 5% conditioner solution. The deposition amount is defined by subtracting the amount of microcapsules in the solution from the first sample jar from the amount of microcapsules in the starting

conditioner composition to be tested. The retention amount is defined by subtracting the amount of microcapsules in the solution from the second sample jar from the deposition amount.

The % Deposition is defined by dividing the deposition amount by the amount of microcapsules in the starting solution. The % Retention is defined by dividing the retention amount by the deposition amount. The %Total Deposition is defined by the % Deposition times the % Retention, divided by 100.

OLFACTIVE GRADING TEST METHOD

The odor performance of a conditioner composition containing polyacrylate microcapsules is evaluated according to the following test method:

a. 0.4 mL of the respective conditioner composition to be tested is applied to a hair switch (IHI, 4 g, 20 cm (8 inches) long, moderately damaged grade) that has been combed, wet, and lightly squeegeed. Lather switch 50-60 strokes (30 seconds) in a milking action.
b. Rinse with stationary shower rinse with no manipulation of hair at (38°C (100 degrees Fahrenheit) water temperature, water flow at 57 L (1.5 gallons) per min, for 30 seconds, water hardness of 8 grains per gallon). Lightly squeegee once down the hair switch from top to bottom between fingers after rinsing to remove excess water.
c. Leave hair to dry at ambient temperature by hanging it on a rack. After approximately 3 hours, olfactively grade the hair switch according to the Primavera Grade (0-100 scale for intensity, where a 10-point difference is consumer noticeable). Record this as the Initial Pre-Comb fragrance intensity.
d. Comb the hair switch 3 times and olfactively grade, record this as the Initial Post-Comb fragrance intensity.
e. Leave the hair switch under ambient conditions (21°C (70 degrees Fahrenheit) and 30% relative humidity) for 24 hours. Then, olfactively grade the hair switch according to the Primavera Grade (0-100 scale for intensity, where a 10-point difference is consumer noticeable), record this as the 24hr aged Pre-Comb olfactive intensity. Comb the hair switch 3 times and assign an olfactive grade, record this as the 24hr aged Post-Comb olfactive intensity.

**EXAMPLES**

The following examples further describe and demonstrate embodiments within the scope of the present invention. The examples are given solely for the purpose of illustration and are not to be construed as limitations of the present invention, as many variations thereof are possible without departing from the spirit and scope of the invention. Where applicable, ingredients are identified by chemical or CTFA name, or otherwise defined below.

The following are examples of polyacrylate microcapsules coated with a block co-polymer falling within the scope of the present invention, as well as comparative examples of polyacrylate microcapsules coated with a block co-polymer not falling within the scope of the present invention. The block co-polymers of Examples C-G, and Comparative Examples A, B and H-N were prepared according to the following synthesis procedure.

BLOCK CO-POLYMER SYNTHESIS

Examples of block co-polymers within the scope of the present invention, and comparative block co-polymers not falling within the scope of the present inventions, were made as follows. For instance, the block co-polymer of Example C of the present invention in Table 1 below was made as follows.

Into a 100 mL round bottom flask were added 5 grams of n-butyl acrylate ("nBA", Molecular Weight 128.17, commercially available from Sigma Aldrich), 0.182 grams of 2-(dodecylthiocarbonothioylthio)-2-methylpropionic acid (commercially available from Sigma Aldrich), 0.0164 grams of 2,2'-azobis(2-methylpropionitrile) (commercially available from Sigma Aldrich) and 40 mL of chlorobenzene (commercially available from Sigma Aldrich). After purging with nitrogen for 20 minutes, the reaction was heated to 65°C for 24 hours. After 24 hours the poly(n-butyl acrylate) was precipitated into cold hexane and dried. The resulting poly(n-butyl acrylate) was characterized by $^1$H NMR in $CDCl_3$ to make sure there is no monomer residual. (Mn = $8.41*10^3$ Daltons, PDI = 1.16).

The poly(n-butyl acrylate) was dissolved in 40 mL chlorobenzene with 7 g of N-dimethylacrylamide (DMAA, commercially available from Sigma Aldrich) and 0.0164 grams of 2,2'-azobis (2-methylpropionitrile) (commercially available from Sigma Aldrich). After purging with nitrogen for 20 minutes, the reaction was heated to 65°C for 24 hours.

The resulting block co-polymer was precipitated into hexane and dried. The block co-polymer is then characterized by $^1$H NMR and GPC (Mn = $2.23*10^4$ Daltons, PDI = 1.182, Molar ratio of DMAA : nBA = 1.75:1).

Additional block co-polymers falling within the scope of the present invention (Examples D-G) and comparative block co-polymers not falling within the scope of the present invention (Comparative Examples A-B and H-N) were prepared via the synthesis method as above for Example C, with the amounts of monomers and reagents adjusted to satisfy the molar ratios specified in Table 1 below for each example.

The number average molecular weight (Mn) of each example was measured according to the MOLECULAR WEIGHT TEST METHOD and reported in Table 1 below. The Water Uptake Value of each block co-polymer example and comparative example was measured according to the WATER UPTAKE VALUE TEST METHOD herein, and are also provided in Table 1 below.

**Table 1**

| Polymer | Molar ratio of Monomers (x:y) | | **Number average molecular weight (Mn) (kDa)** | **Water Uptake (gram of water per gram of polymer)** |
|---|---|---|---|---|
| | Monomer N,N-dimethyl acrylamide (x) by moles of the block co-polymer | Monomer N-butyl acrylate (y) by moles of the block co-polymer | | |
| Comp. A | 2.75 | 1 | 40.6 | <0.1 |
| Comp. B | 1.89 | 1 | 10.0 | <0.1 |
| Ex. C | 1.75 | 1 | 22.3 | 12.89 |
| Ex. D | 2.19 | 1 | 39.3 | 12.19 |
| Ex. E | 2.02 | 1 | 42.0 | 3.49 |
| Ex. F | 2.30 | 1 | 30.8 | 3.55 |
| Ex. G | 1.65 | 1 | 42.0 | 8.08 |
| Comp. H | 1 | 6 | 79.3 | <0.1 |
| Comp. I | 1 | 5 | 67.7 | <0.1 |
| Comp. J | 1 | 2 | 72.8 | <0.1 |
| Comp. K | 1 | 1 | 46.7 | <0.1 |
| Comp. L | 1 | 2 | 118 | <0.1 |
| Comp. M | 1.5 | 1 | 21.7 | <0.1 |
| Comp. N | 1 | 1 | 18.9 | <0.1 |

Additional examples of the block co-polymers are represented in Table 2 below. Such examples were prepared via the synthesis method as above for Example C, with the amounts of monomers and reagents adjusted to provide a molar ratio of DMAA to AA of 2:1 for each example. The number average molecular weight of each example was measured according to the MOLECULAR WEIGHT TEST METHOD and reported in Table 2 below.

**Table 2**

| Polymer | Monomer x | Monomer y | Molar Ratio (x:y) | Number average molecular weight (Mn) (kDa) |
|---|---|---|---|---|
| Ex. O | N,N-dimethyl acrylamide | Ethyl acrylate | 2:1 | 10.5 |
| Ex. P | N,N-dimethyl acrylamide | Propyl acrylate | 2:1 | 12.0 |
| Ex. Q | N,N-dimethyl acrylamide | Hexyl acrylate | 2:1 | 13.5 |
| Ex. R | N,N-dimethyl acrylamide | Dodecyl acrylate | 2:1 | 11.5 |
| Ex. S | N,N-dimethyl acrylamide | Benzyl acrylate | 2:1 | 15.0 |
| Ex. T | N,N-dimethyl acrylamide | Phenyl acrylate | 2:1 | 11.8 |

(continued)

| Polymer | Monomer x | Monomer y | Molar Ratio (x:y) | Number average molecular weight (Mn) (kDa) |
|---|---|---|---|---|
| Ex. U | N,N-dimethyl acrylamide | Ethyl methacrylate | 2:1 | 15.5 |
| Ex. V | N,N-dimethyl acrylamide | Hexyl methacrylate | 2:1 | 13.2 |
| Ex. W | N,N-dimethyl acrylamide | Dodecyl methacrylate | 2:1 | 11.5 |
| Ex. X | N,N-dimethyl acrylamide | Benzyl methacrylate | 2:1 | 10.5 |
| Ex. Y | N,N-dimethyl acrylamide | Phenyl methacrylate | 2:1 | 13.5 |
| Ex. Z | N,N-dimethyl acrylamide | Octadecyl acrylate | 2:1 | 11.7 |

**Table 3**

| Compositions (% wt.) | | | | | | |
|---|---|---|---|---|---|---|
| | Components | Ex.1 | Ex. 2 | Ex. 3 | CEx. i | CEx. ii |
| Group O | Isopropyl alcohol | 0.56 | 0.56 | 0.56 | 0.36 | 0.36 |
| | Behentrimonium methosulfate | 2.26 | 2.26 | 2.26 | 1.42 | 1.42 |
| | Cetyl alcohol | 1.01 | 1.01 | 1.01 | 1.15 | 1.15 |
| | Stearyl alcohol | 2.52 | 2.52 | 2.52 | 2.87 | 2.87 |
| | Benzyl alcohol | 0.4 | 0.4 | 0.4 | 0.4 | 0.4 |
| Group W | Disodium EDTA | 0.13 | 0.13 | 0.13 | 0.13 | 0.13 |
| | Water-soluble preservatives | 0.03 | 0.03 | 0.03 | 0.03 | 0.03 |
| | Deionized Water | q.s. to 100% | | | | |
| Others | Polyacrylate microcapsules *1 | 2 | 6.4 | 6.4 | 2 | 6.4 |
| | Block co-polymer Example C *2 | 0.5*5 | 0.5*5 | 0.5*5 | 0.5*5 | |
| | Deposition polymer-1 *3 | 0.5 | 0.5 | - | - | 0.5 |
| | Deposition polymer-2 *4 | - | - | 0.5 | - | - |

Definitions of Components

*1 polyacrylate microcapsules (TAS1123101 or TAS1122101)

*2 Block co-polymer Ex. C comprising a copolymer of two polymerizable monomers being N,N-dimethyl acrylamide (x = 1.75) and n-butyl acrylate (y = 1) in a molar ratio x:y of 1.75, and the block co-polymer has a number average molecular weight of 22.3 kDa as prepared as set out hereinbefore.

*3 Deposition polymer-1: Copolymer of 30 mass% of acrylic acid monomer and 70 mass% of methoxy-PEG-4methacrylate monomer based on the total mass of the copolymer, and having a weight average molecular weight of 6 300.

*4 Deposition polymer-2: Terpolymer of 40 mass% of methacrylic acid monomer, 55 mass% of methoxy-PEG-4methacrylate monomer, 5 mass% of methoxy-PEG-23methacrylate monomer based on the total mass of the terpolymer, and having a molecular weight of 11000.

*5 0.5% by weight of the solid polyacrylate microcapsules.

Method of Preparation

The above hair conditioner compositions of "Ex. 1" through "Ex. 3" and "CEx. i" through "CEx. ii" were prepared by the following method:
Group O components are mixed and heated to from 66°C to 85°C to form an oil phase. Separately, Group W components are mixed and heated to from 20°C to 48°C to form an aqueous phase. In Becomix® direct injection rotor-stator homogenizer, the oil phase is injected and it takes 0.2 second or less for the oils phase to reach to a high shear field having an energy density of from $1.0x10^5$ $J/m^3$ to $1.0x10^7$ $J/m^3$ where the aqueous phase is already present. A gel matrix is formed. When applicable, the deposition polymer is added to the gel matrix with agitation. Then polyacrylate microcapsules modified or not with a block co-polymer are added to the gel matrix with agitation. Finally, the composition is cooled down to room temperature.

**EX-1: Polyacrylate Microcapsules, small particle size (TAS1123101)**

An oil solution, consisting of 75 g Fragrance Oil Scent A, 75 g of Isopropyl Myristate, 1.0 g DuPont Vazo-52, and 0.8 g DuPont Vazo-67, is added to a 35°C temperature controlled steel jacketed reactor, with mixing at 1000 rpm (4 tip, 2" diameter, flat mill blade) and a nitrogen blanket applied at 100 mL/min. The oil solution is heated to 75°C in 45 min, held at 75°C for 45 min, and cooled to 60°C in 75 min.
A second oil solution, consisting of 37.5 g Fragrance Oil, 1.5 g tertiarybutylaminoethyl methacrylate, 1.2 g 2-carboxyethyl acrylate, and 60 g Sartomer CN975 (hexafunctional urethane-acrylate oligomer) is added when the first oil solution reached 60°C. The combined oils are held at 60°C for an additional 10 min.
Mixing is stopped and a water solution, consisting of 568 g of 25% active Colloid 351, and 282 g of water, is added to the bottom of the oil solution, using a funnel.
Mixing is again started, at 2500 rpm, for 60 min to emulsify the oil phase into the water solution. After milling is completed, mixing is continued with a 3" propeller at 350 rpm. The batch is held at 60°C for 45 min, the temperature is increased to 75°C in 30 min, held at 75°C for 4 hours, heated to 90°C in 30 min and held at 90°C for 8 hours. The batch is then allowed to cool to room temperature.
The finished microcapsules have a median particle size of 1.4 microns, a broadness index of 1.2, and a zeta potential of negative 60 milivolts.

**EX-1 bis: Polyacrylate Microcapsules, large particle size (TAS1122101)**

Capsules are made using identical materials, compositions, and process conditions as in EX-1 with the following exceptions: 1 g of Vazo-52, 0.8 g of Vazo-67, 0.3 g of tertiarybutylaminoethyl methacrylate, 0.25 g of 2-carboxyethyl acrylate, and 12 g of Sartomer CN975 as compositional differences in the oil phase; and 22 g of 25% active Colloid 351, and 308 g of water as compositional differences in the water phase. All other mixing and process conditioners remain the same.
The finished microcapsules have a median particle size of 10.7 microns, a broadness index of 1.5, and a zeta potential of negative 60 milivolts.

**EXPERIMENTAL**

Hair Switches

Hair tresses having a width of 2.5 cm and a length of 20 cm.
Available from *International Hair Importers & Products,* Glendale, NY
Mass: 4.0 g ± 0.05g
Characteristics: cysteic acid: 17.4 - 18.1 mmol/g hair; medullated hair, f: 60-80 mm
General population 4G/8 net slightly flattened

PERFORMANCE ASSESSMENT

The following conditioner compositions were prepared (all amounts are in % wt.). A Control A composition comprised 2% wt. of a slurry of polyacrylate microcapsules (EX-10) without any deposition polymer. A Control B composition comprised 2% wt. of a slurry of polyacrylate microcapsules (EX-10) and 0.5% wt. of a deposition polymer-2 (Terpolymer of 40 mass% of methacrylic acid monomer, 55 mass% of methoxy-PEG-4-methacrylate monomer and 5 mass% of methoxy-PEG-23-methacrylate monomer, based on the total mass of the terpolymer). Conditioner compositions Ex. 1-14 comprised 2% wt. of a slurry of polyacrylate microcapsules (EX-10) modified with a block co-polymer

disposed on an outer surface of the polyacrylate microcapsules, however without any deposition polymer-2 added to the conditioner composition. Conditioner compositions Ex. 15-28 comprised 2% wt. of a slurry of polyacrylate microcapsules (EX-10) modified with a block co-polymer disposed on an outer surface of the polyacrylate microcapsules, however with 0.5% wt. of a deposition polymer-2 added to the conditioner composition.

**Table 4**

| Ingredient | Control A (% wt.) | Control B (% wt.) | Exs. 1-14 (% wt.) | Exs. 15-28 (% wt.) |
|---|---|---|---|---|
| Behentrimonium Methosulfate | 2.85% | 2.85% | 2.85% | 2.85% |
| Isopropyl Alcohol | 0.36% | 0.36% | 0.36% | 0.36% |
| Cetyl Alcohol | 1.01% | 1.01% | 1.01% | 1.01% |
| Stearyl Alcohol | 2.53% | 2.53% | 2.53% | 2.53% |
| Disodium EDTA | 0.13% | 0.13% | 0.13% | 0.13% |
| Benzyl Alcohol | 0.40% | 0.40% | 0.40% | 0.40% |
| Kathon CG | 0.03% | 0.03% | 0.03% | 0.03% |
| Water | QS | QS | QS | QS |
| Polyacrylate microcapsules EX-10 (slurry)*1 | 2% | 2% | 2% | 2% |
| Block co-polymer Examples C-G or Comp. Ex. A, B and H-N | None | None | 1.0% wt. by total weight of the solid Polyacrylate microcapsules | 1.0% wt. by total weight of the solid Polyacrylate microcapsules |
| deposition polymer-2*2 | 0 | 0.5% | 0 | 0.5% |

*1: A slurry of polyacrylate microcapsules (EX-10) is obtained from Encapsys (Appleton, Wisconsin, USA) under Reference PDS040115B having a volume weighted median particle size of 11.58 microns, 44.3% solids, 31.34% total oil (perfume and isopropyl myristate), 0.8% polyvinyl alcohol, pH of 4.42, and the microcapsules having a ratio of core material to shell material of 90:10.

*2: Terpolymer of 40 mass% of methacrylic acid monomer, 55 mass% of methoxy-PEG-4-methacrylate monomer and 5 mass% of methoxy-PEG-23-methacrylate monomer based on the total mass of the terpolymer, and with a weight average molecular weight of 11 100.

**EX-2. Conditioner chassis Making**

The following procedure was used to make a 500 g batch of rinse-off conditioner chassis. (14.26 g) 2.85% wt. of Behentrimonium Methosulfate (at a 80% wt. slurry in isopropyl alcohol) were added to (433.05 g) of preheated water at 95°C, in a 1 L stainless steel vessel that was submerged in a water bath at 92°C. The contents of the 1 L vessel were held under agitation at 350 rpm using a IKA mixer, and a turbine agitator. A transparent solution was obtained after 5 min. Then, (5.05 g) 1.01% wt. of cetyl alcohol flakes, and (12.65 g) 2.53% wt. of stearyl alcohol flakes were added to the stainless-steel vessel, with temperature of the contents controlled to 75 - 85°C. Agitation was increased to 500 rpm. After 10 min, the following ingredients were added to the stainless-steel vessel: (0.65 g) 0.13% wt. of Disodium EDTA, (2.0 g) 0.40% wt. of Benzyl Alcohol, and (0.15 g) (0.03% wt.) of Kathon CG preservative (methylchloroisothiazolinone and methylisothiazolinone). The contents were mixed for 2 min. The stainless-steel reactor was then removed from the constant temperature water bath, and then the contents were cooled to 60°C using a cold-water bath. The stainless-steel reactor was placed under a IKA mill.

**Preparation of the Control conditioner composition A comprising a slurry of polyacrylate microcapsules (EX-10) without a block co-polymer and no addition of a deposition polymer**

2% wt. of a slurry of polyacrylate microcapsules EX-10 were added on top of a pre-made conditioner formula with a 2% wt. formula hole of EX-2. The table above lists the masses of the various ingredients. The resulting mixture

was then speed mixed at 1000 rpm for 2 min using a DAFC 400FVZ speed mixer. The conditioner viscosity and microstructure were characterized to assure that the conditioner formulation meets product design specifications.

**Preparation of the Control conditioner composition B comprising a slurry of polyacrylate microcapsules (EX-10) without a block-copolymer and with the addition of a deposition polymer**

2% wt. of a slurry of polyacrylate microcapsules EX-10 were added on top of a pre-made conditioner formula comprising 0.5% wt. of deposition polymer-2 and a 2.5% wt. formula hole of EX-2. The table above lists the masses of the various ingredients. The resulting mixture was then speed mixed at 1000 rpm for 2 min using a DAFC 400FVZ speed mixer. The conditioner viscosity and microstructure were characterized to assure that the conditioner formulation meets product design specifications.

**Preparation of the conditioner composition Exs. 1-14 comprising a slurry of polyacrylate microcapsules (EX-10) modified with a block co-polymer and no addition of a deposition polymer**

The slurry of polyacrylate microcapsules EX-10 was modified by adding a block co-polymer chosen among one of the block co-polymer Examples A-N. 50 g of the polyacrylate microcapsule slurry EX-10 and 0.222 g of the block co-polymer to be tested is weighed into a glass jar. The jar is capped, shaken vigorously by hand, and then mixed for several hours in a conventional shaker at room temperature. The amount of the block co-polymer added to the slurry of polyacrylate microcapsules EX-10 was 1.0% wt. by weight of the solid polyacrylate microcapsules. 2% wt. of a slurry of polyacrylate microcapsules EX-10 were added on top of a pre-made conditioner formula with a 2% wt. formula hole of EX-2. The table above lists the masses of the various ingredients. The resulting mixture was then speed mixed at 1000 rpm for 2 min using a DAFC 400FVZ speed mixer. The conditioner viscosity and microstructure were characterized to assure that the conditioner formulation meets product design specifications.

**Preparation of the conditioner composition Exs. 15-28 comprising a slurry of polyacrylate microcapsules (EX-10) modified with a block co-polymer and with the addition of a deposition polymer**

The slurry of polyacrylate microcapsules EX-10 was modified by adding a block co-polymer chosen among one of the block co-polymer Examples A-N. 50 g of the polyacrylate microcapsule slurry EX-10 and 0.222 g of the block co-polymer to be tested is weighed into a glass jar. The jar is capped, shaken vigorously by hand, and then mixed for several hours in a conventional shaker at room temperature. The amount of the block co-polymer added to the slurry of polyacrylate microcapsules EX-10 was 1.0% wt. by weight of the solid polyacrylate microcapsules. 2% wt. of a slurry of polyacrylate microcapsules EX-10 were added on top of a pre-made conditioner formula comprising 0.5% wt. of deposition polymer-2 and a 2.5% wt. formula hole of EX-2. The table above lists the masses of the various ingredients. The resulting mixture was then speed mixed at 1000 rpm for 2 min using a DAFC 400FVZ speed mixer. The conditioner viscosity and microstructure were characterized to assure that the conditioner formulation meets product design specifications.

**Deposition of microcapsules onto hair**

The amount of microcapsules deposited onto hair for control A, control B, Ex. 1-28 was assessed according to the DEPOSITION OF MICROCAPSULES DEPOSITED ONTO HAIR TEST METHOD as described hereinabove.

The results of the amount of microcapsules deposited on hair are summarized in the Table 5 below:

**Table 5**

| **Example Reference** | Block co-polymer Example | %Total Deposition on Hair<br><br>no deposition polymer-2 | **Example Reference** | Block co-polymer Example | %Total Deposition on Hair<br><br>with deposition polymer-2 |
|---|---|---|---|---|---|
| Control A | None | < 3.0 | Control B | None | < 8.0 |
| Ex. 1 | Comparative Ex. A | < 3.0 | Ex. 15 | Comparative Ex. A | < 8.0 |
| Ex. 2 | Comparative Ex. B | < 3.0 | Ex. 16 | Comparative Ex. B | |
| Ex. 3 | Ex. C | 25.6 | Ex. 17 | Ex. C | 28.0 |
| Ex. 4 | Ex. D | 27.5 | Ex. 18 | Ex. D | |
| Ex. 5 | Ex. E | 17.8 | Ex. 19 | Ex. E | 20.0 |
| Ex. 6 | Ex. F | 18.7 | Ex. 20 | Ex. F | |
| Ex. 7 | Ex. G | 29.2 | Ex. 21 | Ex. G | 30.5 |
| Ex. 8 | Comparative Ex. H | < 3.0 | Ex. 22 | Comparative Ex. H | < 8.0 |
| Ex. 9 | Comparative Ex. I | < 3.0 | Ex. 23 | Comparative Ex. I | |
| Ex. 10 | Comparative Ex. J | < 3.0 | Ex. 24 | Comparative Ex. J | |
| Ex. 11 | Comparative Ex. K | < 3.0 | Ex. 25 | Comparative Ex. K | |
| Ex. 12 | Comparative Ex. L | < 3.0 | Ex. 26 | Comparative Ex. L | < 8.0 |
| Ex. 13 | Comparative | < 3.0 | Ex. 27 | Comparative | |
| | Ex. M | | | Ex. M | |
| Ex. 14 | Comparative Ex. N | < 3.0 | Ex. 28 | Comparative Ex. N | |

Conclusion

The results provided in Table 5 above demonstrate that polyacrylate microcapsules coated with the block co-polymer falling within the scope of the present invention exhibit improved deposition versus uncoated polyacrylate microcapsules or polyacrylate microcapsules coated with comparative block co-polymer that are not falling within the scope of the present invention.

The results provided in Table 5 above show that the deposition is improved when the polyacrylate microcapsules are coated with the block co-polymer having the right x and y molar ratio, and the right number average molecular weight. Especially, the deposition of the polyacrylate microcapsules onto hair can be improved when the molar ratio of monomer units represented by x to monomer units represented by y is from 1.6:1 to 2.5:1 and not a molar ratio of 2.75:1 or 1.5:1 or 1:1 or 1:6 as for the respective Comp. A, M, N and H (see Table 1). Especially, the deposition of the polyacrylate microcapsules onto hair can be improved when the block co-polymer has a number average molecular weight of at least 11 kDa and not 10 kDa as for the respective Comp. B (see Table 1).

The data illustrates that the modification of the polyacrylate microcapsules coated with the block co-polymer falling within the scope of the present invention has improved the deposition of the polyacrylate microcapsules onto hair when comparing Exs. 3-7 to Control A and versus the block co-polymer not falling within the scope of the present invention such as Exs. 1, 2 and 8-14.

Now, when a deposition polymer is further added to conditioner composition, the combination of the polyacrylate microcapsules modified by a block co-polymer falling within the scope of the present invention and the addition of a deposition polymer in a conditioner composition as described hereinabove (Exs. 17, 19 and 21) can help to further improve the deposition of microcapsules onto hair, versus a control conditioner composition Control B comprising only unmodified polyacrylate microcapsules with the addition of a deposition polymer or versus Ex. 3, 5 and 7 comprising only polyacrylate microcapsules modified by a block co-polymer falling within the scope of the present invention, however without any addition of a deposition polymer.

Polyacrylate microcapsules modified by a block co-polymer disposed on an outer surface of the polyacrylate microcapsules, when the block co-polymer has a formula as recited in the claims, and wherein said block co-polymer has a number average molecular weight of at least 11 kDa as set out hereinbefore, and the addition of a deposition polymer to the conditioner composition can provide a synergistic effect on improving the deposition of polyacrylate microcapsules onto hair. The deposition polymer as described hereinbefore can render the conditioner composition, preferably the gel matrix of the conditioner composition more hydrophobic and bigger in terms of size. Then, the polyacrylate microcapsules modified by the block co-polymer are more viscoelastic. Consequently, the polyacrylate microcapsules modified by the block co-polymer can strongly adhere to the hydrophobic conditioner composition, preferably the gel matrix to be retained onto the hair surface after treating the hair with the conditioner composition.

**Olfactive grading of deposited microcapsules on hair**

The long-lasting odor benefits of the resulting combination of the polyacrylate microcapsules modified with a block co-polymer and the addition of a deposition polymer-2 (Ex. 30) on hair, versus control comprising unmodified polyacrylate microcapsules and deposition polymer-2 (Composition Control B), and versus a conditioner composition comprising only polyacrylate microcapsules modified with a block co-polymer with no deposition polymer-2 (Ex. 29) were evaluated by the OLFACTIVE GRADING TEST METHOD hereinabove.

For these experiments, the block co-polymer of Example G was used as coating for polyacrylate microcapsules as follows. A slurry of polyacrylate microcapsules was obtained from Encapsys (Appleton, Wisconsin, USA) under Reference ID PDS061814A having a volume weighted median particle size of 6.28 microns, 37.24% solids, 26.35% total oil (perfume and isopropyl myristate), 0.8% polyvinyl alcohol, pH of 4.43, and the microcapsules having a ratio of core material to shell material of 90:10.

50 g of the polyacrylate microcapsule slurry and 0.222 g of the block co-polymer of Example G to be tested were weighed into a glass jar. The jar was capped, shaken vigorously by hand, and then mixed for several hours in a conventional shaker at room temperature. The resulting block co-polymer-coated polyacrylate microcapsules comprises 1.0%, by total weight of the solid polyacrylate microcapsules, of block co-polymer.

The Results of the test are summarized in the Table 6 below:

**Table 6**

| **Example Reference** | Block co-polymer Example | Olfactive Grading at 24 hours (Pre/Post Comb) no deposition polymer-2 |
|---|---|---|
| Control A | None | 10/20 |
| Ex. 29 | Ex. G | 10/50 |

| **Example Reference** | Block co-polymer Example | Olfactive Grading at 24 hours (Pre/Post Comb) with deposition polymer-2 |
|---|---|---|
| Control B | None | 10/40 |
| Ex. 30 | Ex. G | 10/60 |

Conclusion

The data illustrates that the combination of the polyacrylate microcapsules with a deposition polymer in a conditioner composition as described hereinabove provides a significant long-lasting odor benefit in use versus the use of only polyacrylate microcapsules in a conditioner composition. More surprisingly, the combination of the polyacrylate microcapsules modified with a block co-polymer and with a deposition polymer in a conditioner composition as described hereinabove provides an even more significant long-lasting odor benefit in use versus the use of only unmodified polyacrylate microcapsules in a conditioner composition

Polyacrylate microcapsules modified by a block co-polymer disposed on an outer surface of the polyacrylate microcapsules, as defined hereinbefore and the addition of a deposition polymer to the conditioner composition can provide a synergistic effect on improving the olfactive grading of deposited polyacrylate microcapsules onto hair at 24 hours.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A conditioner composition comprising:

(a) from 0.004% to 10% of polyacrylate microcapsules by weight of the conditioner composition, wherein the polyacrylate microcapsules comprise an outer surface, wherein the polyacrylate microcapsules comprise a shell material encapsulating a core material, said core material being disposed within said shell material, wherein said shell material comprises a polyacrylate polymer and said core material comprises a benefit agent; and wherein a block co-polymer is disposed on the outer surface of the polyacrylate microcapsules, and wherein said block co-polymer has a formula:

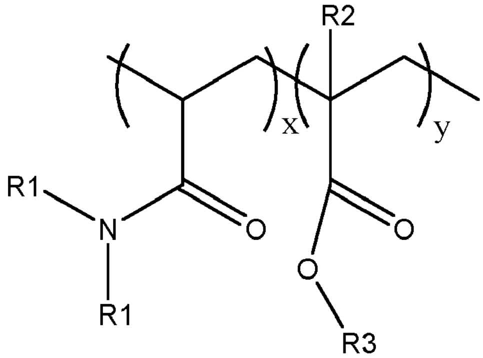

wherein

x and y are integers independently selected such that a molar ratio of monomer units represented by x to monomer units represented by y is from 1.6:1 to 2.5:1;
each R1 is independently selected from the group consisting of H and $CH_3$;
each R2 is independently selected from the group consisting of H and $CH_3$; and
each R3 is independently a $C_1$-$C_{18}$ alkyl group;

wherein said block co-polymer has a number average molecular weight of at least 11 kDa according to the Molecular Weight Test Method as disclosed herein;
(b) from 0.05% to 8% of a deposition polymer by weight of the conditioner composition, wherein the deposition polymer is a copolymer comprising: a vinyl monomer (A) with a carboxyl group in the structure; and a vinyl monomer (B) expressed by the following formula (1):

$$CH_2=C(R^1)\text{-}CO\text{-}X\text{-}(Q\text{-}O)_r\text{-}R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q-O)_r-R^2$, the number of atoms bonded in a straight chain is 70 or less; and wherein the vinyl monomer (A) is contained at a level of from 10 mass% to 50 mass% based on the total mass of the copolymer, and the vinyl monomer (B) is contained at level of from 50 mass% to 90 mass% based on the total mass of the copolymer; and
(c) from 0.05% to 40 % of a conditioning agent, preferably from 0.5% to 30% of a conditioning agent, more preferably from 2% to 25% of a conditioning agent by weight of the conditioner composition, wherein the conditioning agent is selected from the group consisting of a cationic surfactant, a high melting point fatty compound, a silicone compound, and combinations thereof; and
(d) a carrier.

**2.** The conditioner composition of claim 1, wherein the polyacrylate polymer comprises a cross-linked polyacrylate polymer.

**3.** The conditioner composition according to any of the preceding claims, wherein the polyacrylate polymer comprises a polymer derived from a material comprising a multifunctional acrylate moiety selected from the group consisting of tri-functional acrylate, tetra-functional acrylate, penta-functional acrylate, hexa-functional acrylate, hepta-functional acrylate, and mixtures thereof.

**4.** The conditioner composition according to any of the preceding claims, wherein the polyacrylate polymer comprises a moiety selected from the group consisting of an amine acrylate moiety, a methacrylate moiety, a carboxylic acid acrylate moiety, a carboxylic acid methacrylate moiety, and combinations thereof.

**5.** The conditioner composition according to any of the preceding claims, wherein the shell material further comprises from 0.5% to 40%, by weight of the shell material, of polyvinyl alcohol.

**6.** The conditioner composition according to any of the preceding claims, wherein said block co-polymer has a number average molecular weight of from 11 to 45 kDa, preferably from 15 to 43 kDa, or preferably from 20 to 42 kDa according to the Molecular Weight Test Method as disclosed herein.

**7.** The conditioner composition according to any of the preceding claims, wherein x and y are integers independently selected such that the molar ratio of monomer units represented by x to monomer units represented by y is from 2.5 to 1, preferably from 2.0 to 1, or preferably from 1.6 to 1.

**8.** The conditioner composition according to any of the preceding claims, wherein R3 is a $C_1$-$C_8$ alkyl group, preferably a $C_1$-$C_4$ alkyl group; more preferably wherein R1 is $CH_3$ and R2 is H.

**9.** The conditioner composition according to any of the preceding claims, wherein
x and y are integers independently selected such that the molar ratio of monomer units represented by x to monomer units represented by y is 1.65;

R1 is $CH_3$;
R2 is H; and
R3 is a $C_3$ alkyl group.

**10.** The conditioner composition according to any of the preceding claims, wherein the block co-polymer is present in an amount of from 0.01% to 8%, preferably from 0.05% to 5%, more preferably from 0.1% to 3%, even more preferably from 0.5% to 1.5%, by weight of the solid polyacrylate microcapsules.

**11.** The conditioner composition according to any of the preceding claims, wherein the conditioner composition comprises from 1% to 3.5% of a cationic surfactant by weight of the conditioner composition; from 2% to 10% of a high melting point fatty compound by weight of the conditioner composition; and optionally from 0.1% to 8% of a silicone compound by weight of the conditioner composition.

**12.** The conditioner composition according to any of the preceding claims, wherein the vinyl monomer (A) is expressed by the following formula (2) or the following formula (3):

$$CH_2=C(R^3)-CO-(O-(CH_2)_m-CO)_n-OH \qquad (2)$$

wherein $R^3$ represents a hydrogen atom or a methyl group, m represents an integer of 1 to 4, and n represents an integer of 0 to 4;

$$CH_2=C(R^4)-COO-(CH_2)_p-OOC-(CH_2)_q-COOH \qquad (3)$$

wherein $R^4$ represents a hydrogen atom or a methyl group, p and q independently represent an integer of 2 to 6.

**13.** The conditioner composition according to any of the preceding claims, wherein the deposition polymer is a terpolymer comprising: a vinyl monomer (A) with a carboxyl group in the structure; a vinyl monomer (B) expressed by the following formula (1):

$$CH_2=C(R^1)-CO-X-(Q-O)_r-R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q-O)_r-R^2$, the number of atoms bonded in a straight chain is 70 or less;
a vinyl monomer (B1) expressed by the following formula (4):

$$CH_2=C(R^1)-CO-X-(Q-O)_r-R^2 \qquad (4)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 50; and X represents an oxygen atom or an NH group; and, in the following structure $-(Q-O)_r-R^2$, the number of atoms bonded in a straight chain is 250 or less;
and wherein the vinyl monomer (A) is contained at a level of from 10 mass% to 40 mass% based on the total mass of the copolymer, the vinyl monomer (B) is contained at level of from 50 mass% to 89 mass% based on the total mass of the copolymer; and
the vinyl monomer (B1) is contained at level of from 1 mass% to 10 mass% based on the total mass of the copolymer.

**14.** A method of making a conditioner composition, said method comprising, preferably in that order, the steps of:

a) adding a deposition polymer to a conditioning agent to form a pre-conditioner composition, wherein the deposition polymer is a copolymer comprising: a vinyl monomer (A) with a carboxyl group in the structure; and a vinyl monomer (B) expressed by the following formula (1):

$$CH_2=C(R^1)-CO-X-(Q-O)_r-R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q-O)_r-R^2$, the number of atoms bonded in a straight chain is 70 or less; and wherein the vinyl monomer (A) is contained at a level of from 10 mass% to 50 mass% based on the total mass of the copolymer, and the vinyl monomer (B) is contained at level of from 50 mass% to 90 mass% based on the total mass of the copolymer,

wherein the conditioning agent is selected from the group consisting of a cationic surfactant, a high melting point fatty compound, a silicone compound, and combinations thereof; and a carrier;

b) adding polyacrylate microcapsules wherein a block co-polymer is disposed on an outer surface of the polyacrylate microcapsules, and wherein said block co-polymer has a formula:

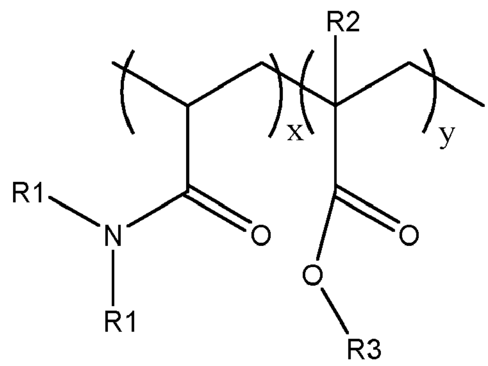

wherein

x and y are integers independently selected such that a molar ratio of monomer units represented by x to monomer units represented by y is from 1.6:1 to 2.5:1;
each R1 is independently selected from the group consisting of H and $CH_3$;
each R2 is independently selected from the group consisting of H and $CH_3$; and
each R3 is independently a $C_1$-$C_{18}$ alkyl group;

wherein said block co-polymer has a number average molecular weight of at least 11 kDa according to the Molecular Weight Test Method as disclosed herein; to the resulting pre-conditioner composition of step (a).

**15.** Use of a deposition polymer in a conditioner composition comprising polyacrylate microcapsules, a conditioning agent and a carrier; for increasing the deposition of polyacrylate microcapsules onto hair for a period of at least 4 hours, preferably from 4 to 24 hours; or for providing a relatively long-lasting odor benefit for a period of at least 4 hours, preferably for a period of at least 24 hours, more preferably from 4 to 24 hours; wherein the deposition polymer is the range from 0.05% to 8% by total weight of the conditioner composition, wherein the deposition polymer comprises a copolymer comprising: a vinyl monomer (A) with a carboxyl group in the structure; and a vinyl monomer (B) expressed by the following formula (1):

$$CH_2=C(R^1)-CO-X-(Q-O)_r-R^2 \qquad (1)$$

wherein: $R^1$ represents a hydrogen atom or a methyl group; $R^2$ represents a hydrogen atom or an alkyl group having a carbon number of 1 to 5, each of which may have a substitution group; Q represents an alkylene group having a carbon number of 2 to 4, which may also have a substitution group; r represents an integer of 2 to 15; and X represents an oxygen atom or an NH group; and, in the following structure $-(Q-O)_r-R^2$, the number of atoms bonded in a straight chain is 70 or less; and wherein the vinyl monomer (A) is contained at a level of from 10 mass% to 50 mass% based on the total mass of the copolymer, and the vinyl monomer (B) is contained at level of from 50 mass% to 90 mass% based on the total mass of the copolymer; and

wherein a block co-polymer is disposed on an outer surface of the polyacrylate microcapsules, and wherein said block co-polymer has a formula:

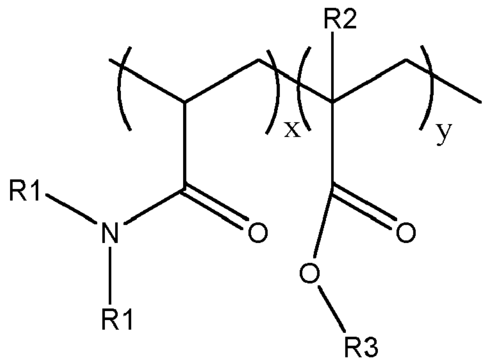

wherein

x and y are integers independently selected such that a molar ratio of monomer units represented by x to monomer units represented by y is from 1.6:1 to 2.5:1;
each R1 is independently selected from the group consisting of H and $CH_3$;
each R2 is independently selected from the group consisting of H and $CH_3$; and
each R3 is independently a $C_1$-$C_{18}$ alkyl group;

wherein said block co-polymer has a number average molecular weight of at least 11 kDa according to the Molecular Weight Test Method as disclosed herein.

Europäisches Patentamt
European Patent Office
Office européen des brevets

# EUROPEAN SEARCH REPORT

Application Number
EP 18 20 2205

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2015/157550 A1 (DIHORA JITEN ODHAVJI [US] ET AL) 11 June 2015 (2015-06-11) * paragraphs [0005] - [0007], [0021] - [0023], [0027] - [0039], [0040], [0047] - [0052], [0064] - [0084]; examples * ----- | 1-15 | INV. A61K8/34 A61K8/41 A61Q5/12 A61K8/81 A61K8/11 |
| Y | WO 2015/047826 A1 (PROCTER & GAMBLE [US]) 2 April 2015 (2015-04-02) * page 1, line 26 - page 2, line 21 * * page 9, line 7 - page 13, line 17 * * examples * ----- | 1-15 | |
| Y | US 2018/265827 A1 (OH HIROSHI [US] ET AL) 20 September 2018 (2018-09-20) * paragraphs [0004] - [0007], [0016] - [0020], [0071] - [0083] * * paragraphs [0084] - [0107], [0237] - [0243]; tables 3,4 * * paragraphs [0253] - [0255], [0258]; table 13 * * paragraphs [0262], [0263]; claim 18; table 14; compound G13 * ----- | 1-15 | TECHNICAL FIELDS SEARCHED (IPC) |
| A | US 2012/276175 A1 (DIHORA JITEN ODHAVJI [US] ET AL) 1 November 2012 (2012-11-01) * the whole document * ----- | 1-15 | A61K A61Q |
| A | US 2013/236411 A1 (ISHIKUBO AKIRA [JP] ET AL) 12 September 2013 (2013-09-12) * table 2 * ----- | 1-15 | |
| T | WO 2018/211485 A1 (PROCTER & GAMBLE [US]) 22 November 2018 (2018-11-22) * the whole document * ----- | 1-15 | |

The present search report has been drawn up for all claims

1

| Place of search | Date of completion of the search | Examiner |
|---|---|---|
| Munich | 28 March 2019 | Steffen, Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 20 2205

This annex lists the patent family members relating to the patent documents cited in the above-mentioned European search report. The members are as contained in the European Patent Office EDP file on
The European Patent Office is in no way liable for these particulars which are merely given for the purpose of information.

28-03-2019

| Patent document cited in search report | Publication date | Patent family member(s) | Publication date |
|---|---|---|---|
| US 2015157550 A1 | 11-06-2015 | CN 103458871 A | 18-12-2013 |
| | | EP 2694031 A2 | 12-02-2014 |
| | | JP 2014510140 A | 24-04-2014 |
| | | JP 2016175935 A | 06-10-2016 |
| | | US 2012282309 A1 | 08-11-2012 |
| | | US 2015157550 A1 | 11-06-2015 |
| | | WO 2012138690 A2 | 11-10-2012 |
| WO 2015047826 A1 | 02-04-2015 | CN 105899183 A | 24-08-2016 |
| | | EP 3052073 A1 | 10-08-2016 |
| | | JP 2016531873 A | 13-10-2016 |
| | | US 2015093347 A1 | 02-04-2015 |
| | | WO 2015047826 A1 | 02-04-2015 |
| US 2018265827 A1 | 20-09-2018 | US 2018265827 A1 | 20-09-2018 |
| | | WO 2018169898 A1 | 20-09-2018 |
| US 2012276175 A1 | 01-11-2012 | CN 103458859 A | 18-12-2013 |
| | | EP 2694017 A2 | 12-02-2014 |
| | | JP 6283607 B2 | 21-02-2018 |
| | | JP 2014510142 A | 24-04-2014 |
| | | JP 2016128499 A | 14-07-2016 |
| | | US 2012276175 A1 | 01-11-2012 |
| | | WO 2012138710 A2 | 11-10-2012 |
| US 2013236411 A1 | 12-09-2013 | BR 112013009836 A2 | 05-07-2016 |
| | | CA 2815351 A1 | 26-04-2012 |
| | | CN 103179949 A | 26-06-2013 |
| | | EP 2630949 A1 | 28-08-2013 |
| | | ES 2654347 T3 | 13-02-2018 |
| | | JP 5998445 B2 | 28-09-2016 |
| | | JP 2012106985 A | 07-06-2012 |
| | | MX 347661 B | 08-05-2017 |
| | | US 2013236411 A1 | 12-09-2013 |
| | | WO 2012053598 A1 | 26-04-2012 |
| WO 2018211485 A1 | 22-11-2018 | EP 3403640 A1 | 21-11-2018 |
| | | US 2018333341 A1 | 22-11-2018 |
| | | WO 2018211485 A1 | 22-11-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62206971 A
- US 20110268802 A
- US 9186642 B
- US 20110269657 A1
- US 9221028 B
- US 20110268778 A1
- US 9162085 B
- US 4275055 A, Nachtigal
- EP 17171644 A
- US 5879584 A
- US 5691297 A
- US 5574005 A
- US 5569645 A
- US 5565422 A
- US 5516448 A
- US 5489392 A
- US 5486303 A

### Non-patent literature cited in the description

- International Cosmetic Ingredient Dictionary. 1993
- CTFA Cosmetic Ingredient Handbook. 1992
- *Dow Corning Corporate Test Method CTM0004,* 20 July 1970